(19) Europäisches Patentamt
European Patent Office
Office européen des brevets

(11) EP 1 413 555 A1

(12) # EUROPEAN PATENT APPLICATION
published in accordance with Art. 158(3) EPC

(43) Date of publication:
**28.04.2004 Bulletin 2004/18**

(21) Application number: **02741360.8**

(22) Date of filing: **28.06.2002**

(51) Int Cl.[7]: **C02F 1/70**, C02F 1/30, C02F 1/32, B08B 3/08, H01L 21/304, C12N 9/08

(86) International application number:
**PCT/JP2002/006560**

(87) International publication number:
**WO 2003/002466 (09.01.2003 Gazette 2003/02)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **29.06.2001 JP 2001200001**
**08.02.2002 JP 2002032845**
**26.04.2002 JP 2002125986**

(71) Applicant: **MIZ Co., Ltd.**
**Fujisawa-shi Kanagawa 251-0871 (JP)**

(72) Inventors:
- **YANAGIHARA, Tomoyuki**
**Fujisawa-shi, Kanagawa 251-0871 (JP)**
- **SATOH, Bunpei**
**Fujisawa-shi, Kanagawa 251-0871 (JP)**
- **SHUDO, Tatsuya**
**Fujisawa-shi, Kanagawa 251-0871 (JP)**

(74) Representative: **Wiebusch, Manfred**
**TER MEER STEINMEISTER & PARTNER GbR,**
**Patentanwälte,**
**Artur-Ladebeck-Strasse 51**
**33617 Bielefeld (DE)**

(54) **METHOD FOR ANTIOXIDATION AND ANTIOXIDATIVE FUNCTIONAL WATER**

(57) A method of antioxidation and antioxidant-functioning water that can transform an antioxidation subject that is in an oxidation state due to a deficiency of electrons, or for which protection from oxidation is desired, into a reduced state where electrons are satisfied, by promoting the breaking reaction of molecular hydrogen that is used as a substrate included in hydrogen-dissolved water into a product of active hydrogen through a process employing a catalyst on the hydrogen- dissolved water, while anticipating high benchmarks of safety on the human body and reduced environmental burden.

FIG. 4

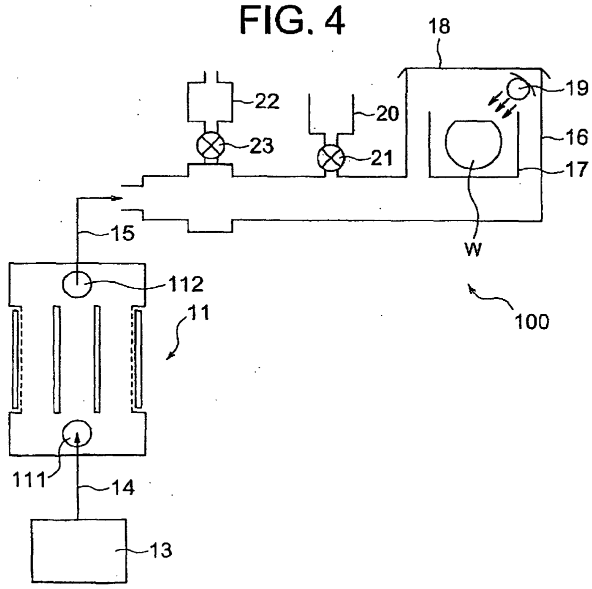

## Description

TECHNICAL FIELD

The present invention relates to a method of antioxidation and antioxidant- functioning water that can transform an antioxidation target that is in an oxidation state due to a deficiency of electrons, or for which protection from oxidation is desired, into a reduced state where electrons are satisfied, by promoting the breaking reaction of a molecular hydrogen substrate included in hydrogen- dissolved water into a product of active hydrogen via a process employing a catalyst on the hydrogen- dissolved water.

BACKGROUND ART

For living organisms, oxygen is a double- edged sword. It has been pointed out that while oxygen is used to procure energy by oxidizing nutrients and perform various oxygen- added reactions essential for living organisms, there is a risk that leads to various types of constitutional disturbances emanating from such oxidizing power.

In particular, it is known that a metabolism- produced active oxygen species called superoxide anion radical ($O_2^{-}\cdot$) is reduced through a metal catalyst such as iron or copper to become hydrogen peroxide ($H_2O_2$) and then becomes a highly reactive hydroxyl radical (-OH) that denatures protein and breaks the chain of DNA. In addition, these active oxygen species(($O_2^{-}\cdot$) ($H_2O_2$), ($\cdot$OH),) oxidizes lipids and produces lipid peroxide, a factor that accelerates the aging process.

In living organisms, for example, superoxide anion radical ($O_2^{-}\cdot$) having such toxicity is normally scavenged with an enzyme called superoxide dismutase (SOD).

However, it has been found that if balance in the organism is upset, for example by factors such as stress, alcohol, smoking, strenuous exercise, or aging, SOD levels decrease and lipid peroxide increases because of the active oxygen species. This brings on various health problems such as heart attacks, arteriosclerosis, diabetes, cancer, stroke, cataracts, stiff shoulders, over sensitivity to cold, high blood pressure, and senile dementia, as well as problems such as age spots, freckles, and wrinkles.

Active oxygen scavenging agents and anti-oxidizing agents such as butyl hydroxy anisol (BHA), butyl hydroxy toluene (BHT), alpha- tocopherol, ascorbic acid, cysteine, and glutathione are known as substances for remedying such active oxygen species- derived diseases.

Nevertheless, since such anti-oxidizing agents are chemically synthesized compounds, there are problems including remaining doubts as to the safety of such substances on the human body when used habitually in large quantities. Another problem is the fact that these and similar anti-oxidizing agents become oxidized themselves through the process of reducing other substances and raises questions as to the safety of such by-product oxides on the human body.

Accordingly, development of innovative technology that can anticipate a high benchmark of safety on the human body while demonstrating antioxidation capability and active oxygen species scavenging capability that is on par with or superior to for instance conventional anti-oxidizing agents has been long awaited.

In the meantime, global- scale environmental problems have come under close scrutiny in recent years as a result of, for example, industrial waste, medical waste, and industrial effluent being discharged into the global environment.

For instance, during the manufacturing process for industrial products and medical products, when performing rinsing, etching, post-processing, or the like, processing is performed using a solution including chlorofluorocarbon (CFC) or a halogen such as chlorine, an acidic solution such as hydrochloric acid, an alkaline solution, or gases including a halogen or CFC. More specifically, in the field of rinsing semiconductor wafers, specifically silicon wafers, silicon wafer surface treatment is performed using either deionized water, or a mixed solution including acidic solutions of deionized water and an acid such as hydrochloric acid, hydrofluoric acid, sulfuric acid, nitric acid, or hydrogen peroxide, and alkaline solutions of deionized water and an alkali such as ammonium hydroxide or an organic alkali.

However, when performing rinsing or other treatment using, for example, such chemical solutions, halogenated compounds or CFC compounds are produced creating industrial waste that is difficult to process for disposal, and there is a problem with increased burden on the environment as a result of this intractable industrial waste being discharged into the global environment.

Accordingly, development of innovative technology that can anticipate a high benchmark of reduced environmental burden achieved by not using the above-mentioned or similar chemical solutions or drastically reducing the amount used while maintaining processing results for rinsing, etc., that is on par with or superior to processing using conventional chemical solutions and the like has been long awaited.

The present invention has been made in order to solve such problems and aims to provide a method of antioxidation and antioxidant- functioning water that can transform an antioxidation subject that is in an oxidation state due to a deficiency of electrons, or for which protection from oxidation is desired, into a reduced state where electrons are satisfied, by promoting the breaking reaction of molecular hydrogen that is used as a substrate included in hydrogen-dissolved water into a product of active hydrogen through a process employing a catalyst on the hydrogen- dissolved water, while anticipating high benchmarks of safety on the human body and reduced environmental burden.

DISCLOSURE OF THE INVENTION

Before giving a general description of the invention, the history of how the inventors arrived at the present invention is described.

(1) History of Invention Idea

In the previously filed and published Republished Patent No. WO99/10286, the contents of which are incorporated herein by reference, the applicants of the present application disclose an electrolytic cell and an electrolyzed water generation apparatus capable of independently controlling the hydrogen ion exponent (hereafter referred to as "pH") and the oxidation/ reduction potential (hereafter referred to as "ORP"). A synopsis of the aforementioned application is given hereinforth. Namely, the electrolytic cell and reducing potential water generation apparatus have an electrolytic chamber to which raw water is supplied, and at least a pair of electrode plates provided inside the electrolytic chamber and outside the electrolytic chamber so as to sandwich a membrane, wherein the electrode plates (open system) provided outside the electrolytic chamber is provided in contact with the membrane or leaving a slight space. The electrolytic cell and reducing potential water generation apparatus are also configured with a power source circuit that applies a voltage between both electrodes, wherein the electrode plate provided inside the electrolytic chamber is given as the cathode and the electrode plate provided outside the electrolytic chamber is given as the anode. On the cathode side in the apparatus, without significantly changing the pH of the raw water, electrolyzed reducing potential water (hereafter, also referred to as "reducing potential water") is generated having an ORP that is significantly lowered to a negative value. In the following, unless not specifically stated otherwise, "electrolysis processing" means carrying out continuous- flow electrolysis processing using the above- mentioned reducing potential water generation apparatus under electrolysis conditions of a 5 A constant current and flow rate of 1 L/min.

The inventors herein arrived at the present invention during performance evaluation testing of reducing potential water generated with the reducing potential water generation apparatus described above.

Here, the reducing potential water has a negative ORP value, and also shows an ORP value corresponding to the pH that exceeds a predetermined value. Whether or not the ORP value exceeds the predetermined value may be determined through the following Nernst equation (approximate equation):

$$ORP = -59pH - 80(mV) \qquad \text{(Nernst equation)}$$

As shown in FIG. 1, this equation shows whether there is a proportional relationship between the pH and ORP (the ORP value falls towards negative as the pH falls towards the alkaline side). Here, the fact that the ORP value corresponding to pH shows a value that exceeds the predetermined value means that the ORP value is lower than the value according to the Nernst equation described above. It is given here that water meeting such conditions is called reducing potential water. For example, substituting pH 7 into the Nernst equation above gives an ORP of approximately -493 (mV). In other words, at pH 7, water having an ORP of approximately -493 (mV) or lower corresponds to reducing potential water. However, some difference definitely exists in the dissolved hydrogen concentration within the category of reducing potential water defined here, but this is described later together with the quantitative analysis method for this dissolved hydrogen concentration.

Therefore, a considerable amount of high-energy electrons is included in the reducing potential water. This is clearly seen when measured with an ORP meter. The ORP is an indicator showing the proportions with which oxidizing material and reducing material exist in the test water, and generally uses units of millivolts (mV). Generally with an ORP meter, a negative ORP value is observed when the measurement electrode takes a negative charge, and conversely, a positive ORP value is observed when the measurement electrode takes a positive charge. Here, in order for the measurement electrode to take a negative charge, high- energy electrons must be included in the test water. Accordingly, the fact that ORP value shows a negative value having a large absolute value can be said as meaning that the test water includes high- energy electrons.

At this point, illumination testing using a light emitting diode (hereafter abbreviated as "LED") was carried out for performance evaluation showing to what extent high- energy electrons are included in the reducing potential water. This used the principle behind batteries. More specifically, reducing potential water having an exemplary ORP of approximately -600 (mV) and tap water having an exemplary ORP of approximately +400 were poured into the cathode chambers 205 and anode chambers 207, respectively, in a testing cell 209 configured with alternating platinum or similar electrodes 201 and membranes 203, and having about three cathode chambers and three anode chambers. Continuous illumination of an LED 211 was observed when the minus end of the LED 211 was connected to the electrode in contact with a cathode chamber 205 and the plus end of the LED 211 was connected to an anode chamber 207. This means that current is flowing from the anode of the cell 209 towards the cathode, and moreover, the fact that current is flowing means that electrons are flowing. At this point, taking into consideration the fact that the electrons flowing through the LED 211 are flowing from the cathode of cell 209 to the anode, the included that high- energy electron groups in the reducing potential water are quantitatively evaluated through testing.

As reference examples, alkaline electrolyzed water generated by a commercially available electrolyzed water generation apparatus (exemplary ORP of approximately -50 mV), or natural mineral water, etc, was poured into the cathode chambers and tap water was poured into the anode chambers. However, in this case, continuous illumination of the LED was not observed when the minus end of the LED was connected to the electrode in the cathode chamber and the plus end of the LED is connected to the anode chamber in a manner similar to that described above. This is thought as happening because not enough high- energy electron groups to illuminate the LED are included in the existing alkaline electrolyzed water or natural mineral water.

In addition, even if flow were to be reduced and the ORP value shifted significantly towards the negative with a commercially available electrolyzed water generation apparatus, should the absolute value of the ORP value occurring at the pH level at that time be small in accordance with the above- mentioned Nernst equation, no illumination of the LED would naturally be observed. With for example the commercially available electrolysis generation device, even if the pH is approximately 10 and the ORP value is in the range of -500 to -600 (mV) as a result of reducing the flow, since the ORP value as a percentage of the pH level becomes small, it may be considered as becoming weak in terms of the electron energy, and as long as ORP value fails to be brought down to at least approximately -670 (mV) or lower when the pH level is approximately 10, it is impossible to illuminate the LED.

In addition, there are several varieties of LEDs. In particular, when a diode showing for example a blue or green color that requires a high inter-terminal voltage of approximately 3V or higher was used, continuous illumination of such diode was observed when using a cell 209 having each chamber arranged in a three-layer alternating structure as described above.

Therefore, as eager research progressed on the industrial applicability of having high- energy electrons included in reducing potential water, a hint was received that wondered if it was possible that the reducing potential water had "latent reducing power". In particular, the reducing potential water had quite strong reducing power since the ORP value had fallen to a appreciably negative value that was significant enough cause the LED to illuminate, which led to the feeling that if this reducing power be could tapped there may be applications over a wide range of industrial fields including health care, manufacturing, food, agriculture, automobile, and energy.

What state this "latent reducing power" is in is now described.

For instance, if a reducing agent such as vitamin C (ascorbic acid) is added to ordinary tap water, and thereafter an oxidizing agent is further added, the reducing agent immediately reduces the oxidizing agent. On the other hand, if an oxidizing agent is added to reducing potential water, the oxidizing agent is not immediately reduced at all. Conditions at this point may be considered as including both the significant negative ORP value for the reducing potential water remaining the same, as well as the oxidizing agent also maintaining the same conditions. At this point in time reducing power has not yet been exhibited.

That is, no matter how much the high- energy electrons try to exist in the reducing potential water, or to put it another way, no matter how large and negative the value of the ORP is, it comes up against the fact that the reaction where electrons are immediately released from the reducing potential water to reduce the oxidizing agent does not occur. Therefore, it was thought that the magnitude of the electron energy included in the reducing potential water and how easily the electrons are released or the exhibition of reducing power are probably two separate issues.

So what should be done to make the reducing potential water exhibit reducing power? As the inventors continued with their eager research into this proposition, the idea of using some sort of catalyst hits them with a flash of light. While there is many types of catalysts, with the particular premise of for instance use in living organisms, the idea was conceived that some sort of enzyme or a precious metal catalyst colloid, which is described later, might be used as the catalyst.

Here, the particular mention of an enzyme is for an enzyme-acting substance that is a chemical reaction catalyst, and the activity of the enzyme is measured by the speed of the catalyzing reaction. In the case of catalyzing the reaction of A→B, A is the substrate and B is the product. Applying this to the case of the present invention, the molecular hydrogen included in the hydrogen- dissolved water corresponds to the substrate, and the active hydrogen corresponds to the product. Also, it is thought that the working- action mechanism of such enzyme can be described in the following manner:

It is assumed here that it is necessary for the high- energy electron group included in the reducing potential water to come into contact with the oxidizing agent and reduce this oxidizing agent. There is an energy wall that this electron group included in the reducing potential water must surpass in order for the electron group to migrate to the oxidizing agent. This energy wall is commonly called a "potential barrier", "activation energy", or the like. The higher this energy is, the higher the height of the wall that needs to be surpassed becomes. Also, the energy that can be expressed with the height of this wall is larger than the energy of the electron group; therefore the electron group is normally not able to climb over this wall and as a result does not migrate to the oxidizing agent. In short, it is thought that the oxidizing agent cannot be reduced.

However, the activation energy corresponding to the height of the wall may be lowered if for instance a catalyst such as an enzyme is used. As a result, the electron group included in the reducing potential water is able to migrate to the oxidizing agent rather smoothly compared to when no catalyst is used, and at the endpoint where this migration is complete, the reducing potential water is able to reduce the oxidizing agent.

In this manner, when an enzyme or similar catalyst is used, the high-energy electron group included in the reducing potential water can be more easily released, and results in the reducing power being exhibited. In other words, this is what is meant by the reducing potential water "having latent reducing power", which may be rephrased as "the reducing power held by the reducing potential water is kept under seal". These various thought processes led to the idea that "the key to lifting the seal on the reducing power held by the reducing water is a catalyst."

Now that the history of the idea of the invention has been elucidated, a synopsis of the invention will be described.

(2) Synopsis of Invention

Antioxidation Method

The present invention provides an antioxidation method that includes transforming an antioxidation subject that is in an oxidation state due to a deficiency of electrons, or for which protection from oxidation is desired, into a reduced state where electrons are satisfied, by promoting the breaking (activating) reaction of molecular hydrogen used as a substrate included in hydrogen- dissolved water into a product of active hydrogen via a process employing a catalyst on the hydrogen- dissolved water.

The inventors are confident that the substance that provides the negative value for the ORP value of hydrogen-dissolved water such as electrolyzed water or hydrogen bubbling water is the hydrogen that is dissolved in that water. The fact that hydrogen is the ultimate reducing substance, and furthermore, the fact that hydrogen develops on the cathode side during electrolysis processing serves as proof of this conviction.

Nevertheless, as made clear in the history of the idea behind the invention, with the hydrogen- dissolved water as it is, the reducing power is normally kept under seal.

Therefore, in order to cast off the seal on the reducing power held by the hydrogen- dissolved water, as defined with the antioxidation method according to the present invention, it has been found that the step of using a catalyst in the hydrogen- dissolved water is extremely important.

Another important factor is the existence of an antioxidation subject. If there is no antioxidation subject, then there is no stage for the antioxidation action according to the present invention to be exhibited.

In other words, the important factors in the present invention are 1) the hydrogen- dissolved water, 2) the catalyst, and 3) the antioxidation subject. When these three factors are organically combined, the seal on the reducing power latently held by the hydrogen is cast off to allow manifest expression of the broad antioxidation function including the reducing function. It should be noted that the expression of the antioxidation function spoken of in the present invention is the reduced state where electrons are satisfied in the antioxidation subject that is either in an oxidized state due to a deficiency of electrons or for which protection from oxidation is desired. While magnitude of the reducing power here may be estimated to a certain extent through, for example, the condition of the ORP value (i.e. the stability of the ORP reading or the relationship with the above-mentioned Nernst equation), ultimately it is determined depending on the effective value of the dissolved hydrogen concentration DH found using the dissolved hydrogen concentration quantitative method (described later) that uses an oxidization/ reduction pigment.

Next, the technical scope that is assumed for the present invention regarding these three factors will be laid out.

Hydrogen dissolved water

Hydrogen dissolved water is assumed to be any water in which there is included hydrogen. In addition, what is called water here (also referred to as raw water) includes all waters including tap water, purified water, distilled water, natural water, activated charcoal processed water, ion exchange water, deionized water, ultra pure water, commercially available (PET) bottled water, biological fluid (described later), and water in which molecular hydrogen is generated through a chemical reaction in the water. Furthermore, all water that includes an auxiliary agent for electrolysis or a reducing agent added to such water also falls within the technical scope of the present invention. Moreover, as long as it meets the condition of being water in which there is included hydrogen, it does not matter if the water is acidic, neutral, or alkaline, nor does it particularly matter if the dissolved concentration is high or low. However, since the antioxidation function expressed through application of the present invention emanates from the electrons released through the process of replacing molecular hydrogen with active hydrogen through a catalyst, more significant expression of the antioxidation function may be expected with a higher dissolved concentration of molecular hydrogen.

Moreover, hydrogen- dissolved water also includes either alkaline electrolyzed water generated on the cathode side when raw water is subjected to electrolysis processing between an anode and a cathode via a membrane, or water processed through bubbling or pressurized filling of hydrogen into raw water. The definition is made in this way in order to make clear that "alkaline ion water" that is produced through existing continuous flow-type or batch electrolyzed water generation apparatus as well as hydrogen- dissolved water generated by inclusioning hydrogen in raw water through external manipulation also fall within the technical scope of the present invention. Those given as hydrogen- dissolved water here are merely examples and is not intended to mean that they are limited to this. Accordingly, it should be made clear now that even if using for instance natural water and hydrogen is inclusioned therein, this does not mean that such water falls outside of the technical scope of the present invention.

In addition, molecular hydrogen thought as being generated by enteric microorganisms, particularly microorganisms that contain hydrogenase, is dissolved inside bodily fluids (also referred to as biological fluids) such as the blood or lymphatic fluid of living organisms. Hydrogen dissolved water mentioned in the present invention, regardless of origin, also includes biological fluid in which molecular hydrogen is dissolved, and as such falls within the technical scope thereof. It should be noted that the location of the molecular hydrogen occurring in the living organism does not remain within the intestinal tract, but is also absorbed from the intestines and distributed through blood. This molecular hydrogen that has entered the blood flow is thought to be transported to each of the internal organs such as the liver and kidneys, and stored in the various parts of the body. In this case, the activation of molecular hydrogen should be facilitated by administering an enzyme such as hydrogenase or a precious metal colloid (described later) to the living organism in order to utilize the molecular hydrogen existing in the living organism as a reducing agent.

However, hydrogen- dissolved water also includes reducing potential water where the ORP is a negative value, and the ORP value corresponding to the pH shows a value that is lower than the value according to the Nernst equation or ORP = -59 pH - 80 (mV). The reducing potential water mentioned here naturally includes water generated with the reducing potential water generation apparatus developed by the applicants herein (hereafter simply referred to as the "reducing potential water generation apparatus"), and it should be made clear now that this also includes water that while generated with an apparatus other than such apparatus meets the conditions for reducing potential water described above. It should be now added that in the case of employing a buffered electrolysis processing technique in the reducing potential water generation apparatus wherein water that has been generated is again introduced into the electrolytic cell so as to circulate, and then repeating this circulatory process for a predetermined length of time, as shown for instance in the following Table 1, reducing potential water may be obtained having a high dissolved-hydrogen concentration and an even lower ORP value, and superior reducing power (antioxidizing power) may be expressed with such reducing potential water.

Therefore, the respective physical quantities of reference examples of hydrogen- dissolved water assumed by the inventors and comparative examples of water in which no hydrogen is dissolved are now given. Activated charcoal processing water resulting from processing Fujisawa City tap water through an activated charcoal column, Organo purified water resulting from processing Fujisawa municipal tap water through a ion exchange column made by Organo Corporation, and an example of (PET) bottled water: "evian" (registered trademark of S.A. des Eaux Minerales d' Evian), which is supplied in Japan through Calpis Itochu Mineral Water Co., Ltd., are given as examples of subject water for purposes of comparison. A first reducing potential water subjected to continuous electrolysis processing using electrolysis conditions of a 5 A constant current and flow rate of 1 L/min in the reducing potential water generation apparatus developed by the applicants herein, and a second reducing potential water subjected to continuous buffered electrolysis processing for 30 minutes using the same electrolysis conditions (amount of buffered water was 2 liters) in the same apparatus are given as examples of each type of post-processing hydrogen-dissolved water for the purpose of dissolving hydrogen in such comparative subject waters. In addition, hydrogen gas bubbling water subjected to hydrogen gas bubbling processing for 30 minutes, and alkaline electrolyzed water subjected to continuous electrolysis processing using electrolysis conditions of electrolysis range "4" with a standard amount of water in a "Mini Water" electrolyzed water generation apparatus made by MiZ Co., Ltd. are given as examples vis-à-vis each type of comparative subject water.

Furthermore, pH, oxidizing/ reducing potential ORP (mV), electrical conductance EC (mS/m), dissolved oxygen concentration DO (mg/L), dissolved hydrogen concentration DH (mg/L), and water temperature T (°C) are given as the various physical properties in such waters. In addition, the various types of gages used to measure these physical properties include the following: the pH meter (including a temperature gage) is a model D-13 pH meter made by Horiba, Ltd. with a model 9620-10D probe for the same, the ORP meter is a model D-25 ORP meter made by Horiba, Ltd. with a model 9300- 10D probe for the same, the EC meter is a model D-24 EC meter made by Horiba, Ltd. with a model 9382- 10D probe for the same, the DO meter is a model D-25 DO meter made by Horiba, Ltd. with a model 9520-10D probe for the same, and the DH meter (dissolved hydrogen meter) is a model DHD I-1 made by DKK-TOA Corporation with a model HE-5321 electrode (probe) and model DHM-F2 repeater for the same. The various physical properties of the comparative subject waters were respectively measured using these types of gages.

[Table 1]

BASIC DATA FOR EACH WATER

| PHYSICAL PROPERTIES FOR WATER WITHOUT HYDROGEN INCLUSION | pH | ORP [mV] | EC [mS/m] | DO [mg/L] | DH [mg/L] | T [°C] |
|---|---|---|---|---|---|---|
| ACTIVATED CHARCOAL PROCESSED WATER | 7.31 | 308 | 16.15 | 8.65 | 0.000 | 22.2 |
| ORGANO PURIFIED WATER | 6.00 | 395 | 0.11 | 4.52 | 0.000 | 23.3 |
| evian (REFRIGERATED) | 7.30 | 407 | 56.30 | 9.76 | 0.000 | 12.5 |
| PHYSICAL PROPERTIES WITH ONE-TIME ELECTROLYSIS | pH | ORP [mV] | EC [mS/m] | DO [mg/L] | DH [mg/L] | T [°C] |
| ACTIVATED CHARCOAL PROCESSED WATER | 9.54 | -735 | 22.30 | 3.22 | 0.900 | 27.5 |
| ORGANO PURIFIED WATER (not 5A) | 10.48 | -760 | 5.60 | 4.45 | 0.425 | 24.2 |
| evian (REFRIGERATED) | 7.48 | -530 | 56.10 | 5.25 | 0.460 | 15.7 |
| PHYSICAL PROPERTIES WITH BUFFERED ELECTROLYSIS (30 MIN) | pH | ORP [mV] | EC [mS/m] | DO [mg/L] | DH [mg/L] | T [°C] |
| ACTIVATED CHARCOAL PROCESSED WATER | 11.00 | -850 | 42.80 | 1.76 | 1.332 | 25.8 |
| ORGANO PURIFIED WATER (not 5A) | 11.15 | -850 | 52.30 | 0.94 | 1.374 | 31.9 |
| evian (REFRIGERATED) | 7.72 | -635 | 45.10 | 1.46 | 1.157 | 24.2 |
| PHYSICAL PROPERTIES WITH HYDROGEN GAS BUBBLING (30 MIN) | pH | ORP [mV] | EC [mS/m] | DO [mg/L] | DH [mg/L] | T [°C] |
| ACTIVATED CHARCOAL PROCESSED WATER | 8.30 | -585 | 17.97 | 1.67 | 1.070 | 23.6 |
| ORGANO PURIFIED WATER | 6.40 | -550 | 0.22 | 1.75 | 1.090 | 23.4 |
| evian (REFRIGERATED) | 8.25 | -765 | 50.7 | 2.59 | 0.89 | 21.3 |
| ACTIVATED CHARCOAL PROCESSED WATER (by NaOH) | 11.00 | -836 | 33.50 | 1.55 | 0.910 | 20.9 |
| PHYSICAL PROPERTIES WITH ELECTROLYSIS IN ELECTROLYZED WATER GENERATION APPARATUS | pH | ORP [mV] | EC [mS/m] | DO [mg/L] | DH [mg/L] | T [°C] |
| ALKALINE ELECTROLYZED WATER (NORMALLY EQUIPPED ACTIVATED CHARCOAL) | 9.34 | 60 | 14.78 | 8.00 | 0.163 | 20.7 |

According to this Table 1, focusing on the dissolved hydrogen concentration (DH) measured with the dissolved hydrogen meter, with the first reducing potential water subjected to one-time electrolysis processing using the reducing potential water generation apparatus, despite the fact that the electrolyzed water was instantly removed, it was found that a high concentration of hydrogen ranging between 0.425 and 0.900 (mg/L) was dissolved therein.

In addition, in the case where the length of processing time was for example 30 minutes, comparing the dissolved hydrogen concentrations of the buffered electrolyzed reducing potential water (the second reducing potential water) in this reducing potential water generation apparatus and the hydrogen gas bubbling water, while the latter ranged between 0.89 and 1.090 (mg/L), the former showed that a high concentration of hydrogen ranging between 1.157 and 1.374 (mg/L) could also be dissolved therein.

Meanwhile, it is preferable that at least one reducing agent selected from the group consisting of sulfite, thiosulfate, ascorbic acid, and ascorbate be added as required to the hydrogen- dissolved water. This is because it is preferable that the dissolve oxygen concentration in the hydrogen- dissolved water be made as low as possible when it is necessary to prevent rapid oxidization due to the dissolved oxygen of the active hydrogen occurring through the action of the catalyst.

To further explain this, in hydrogen- dissolved water where a catalyst has been used, it is possible to reduce the dissolved oxygen concentration DO (mg/L) to nearly zero (mg/L) when the amount of reducing agent added is less than the chemical equivalent capable of exactly reducing the dissolved hydrogen.

As a comparative example for this, when the same amount of reducing agent was added to hydrogen- dissolved water where a catalyst had not been used, significant reduction in the dissolved oxygen concentration DO (mg/L) was not achieved. This is thought to be the result of the intrinsic reducing power held by the hydrogen- dissolved water on which the seal had been lifted bringing out the reducing power held by the reducing agent more strongly.

Accordingly, it should be added that in the case of bottling antioxidant- functioning water according to the present invention in the condition where both a reducing agent and a dissolved additive such as a vitamin coexist, there is also the dimension that such an additive causes the antioxidizing action intrinsically held by the additive to be brought out even more strongly as a result of being in an antioxidizing environment. This is because when antioxidant-functioning water according to the present invention is bottled in the condition where both a reducing agent and the exemplary reducing ascorbic acid coexist, it means that the ascorbic acid causes the antioxidizing action intrinsically held by the reducing ascorbic acid to be brought out even more strongly as a result of continuing to be in reducing form due to being in an antioxidizing environment. In this case, it is preferable that the reducing agent such as the exemplary reducing ascorbic acid be added in an amount greater than that required to reduce/neutralize the oxidizing material such as dissolved oxygen in the coexistent system. However, it is preferable that an appropriate amount of additive ascorbic acid be added in consideration of the pH expressed by the antioxidant- functioning water and the minimum recommended daily amount that should be ingested.

Catalyst

The catalyst is assumed to be all those having the function of catalyzing the breaking reaction of the molecular hydrogen used as a substrate included in the hydrogen- dissolved water into a product of active hydrogen. More specifically, the essential qualities of the catalyzing function according to the present invention lies in smoothly accelerating the activation of molecular hydrogen, and within such function, accepting electrons from the molecular hydrogen (by activating one molecular hydrogen, two electrons are obtained or $H_2 \rightarrow 2e^- + 2H^+$) and donating the accepted electrons to the antioxidation subject following temporary pooling (including the idea of absorption or occlusion into the catalyst) or without pooling. The catalyst according to the present invention may be, for example, a hydrogen oxidization/ reduction enzyme. Furthermore, a hydrogenase, a precious metal colloid (described later), or one of the electromagnetic waves selected from the group consisting of visible light, ultraviolet light, and electron beams also falls within the technical scope. It should be noted that the precious metal colloid assumed with the present invention means the inclusion of platinum, palladium, rhodium, iridium, ruthenium, gold, silver, or rhenium, along with the respective salts thereof, alloy chemical compounds, or colloid molecules themselves such as complex chemical compounds, as well as mixtures of these. When making or using these precious metal colloids, reference should be made to the contents of "Fabrication and Use of Pt Colloids (Pt koroido no tsukurikata to tsukaikata)" (NANBA, Seitaro and OKURA, Ichiro); Hyomen Kagaku (Surface Science) Vol. 21; No. 8 (1983), the contents of which are included herein by reference. In addition, the colloid mentioned in the present invention is assumed as having molecules with diameters ranging between 1 nm and 0.5 μm, which is said as showing innate behavior of a general colloid. However, when employing the exemplary Pt colloid as the precious metal colloid, it is considered proper to use a molecular diameter that increases the catalytic activity of this Pt colloid, preferably ranging between 1 and 10 nm and more preferably between 4 and 6 nm. This is, as written in the above-mentioned "Fabrication and Use of Pt colloids" by Nanba and Okura, the molecular size is derived from the trade- off relationship between the fact that the innate property is expressed as a precious metal and the fact that the surface area is increased to improve the catalyst activity. However, the colloids mentioned in the present invention are in accordance with the definition proposed by Staudinger of Germany that "colloids are configured with between $10^3$ and $10^9$ atoms." Moreover, the precious metal colloid according to the present invention preferably has a round molecular shape in order to increase the surface area. Here, since the fact that the surface area of the precious metal colloid is large means increased opportunities for connection with the molecular hydrogen used as the substrate, it is superior from the viewpoint of catalytic function expressed by the precious metal colloid.

Moreover, a catalyst includes the idea of electron carriers such as a coenzyme that assists the functioning thereof, inorganic compounds, and organic compounds.

It is preferable that such an electron carrier have properties capable of efficiently accepting electrons from hydrogen, a hydrogen oxidization/reduction enzyme, a hydrogenase, or a precious metal colloid, which are all electron donors, and at the same time, efficiently carrying electrons to the antioxidation subject, which is an electron acceptor. To put it more simply, the electron carrier acts to transport the hydrogen (electron).

In the following, candidates for the electron carrier are now given. It should be noted that it does not matter if the electron carrier is oxidizing or reducing. Since the reducing electron carrier has surplus electrons beforehand, it is beneficial from the viewpoint of easily releasing electrons.

(1) Methylene blue (normally oxidizing)
methylthionine chloride, tetramethylthionine chloride
chemical formula = C16H18ClN3S · 3(H20)
Reducing methylene blue is referred to as leucomethylene blue.

(2) Pyocyanin
chemical formula = C13H10N2O

One of the antibiotic substance produced by Pseudomonas aeruginosa. Pyocyanin performs reversible oxidization/ reduction reactions, and there are two types of the oxidizing type: one that is alkaline and a blue color, and one that is acidic and a red color. In addition, the reducing type is colorless, as is the reduced methylene blue (leucomethylene blue).

(3) Phenazine methosulfate
abbreviation = PMS
chemical formula = C14H14N2O4S
Phenazine methosulfate tends to easily photo-decompose.

(4) 1 - Methoxy PMS

Is stable when exposed to light and was developed as a substitute for the PMS mentioned above that is unstable when exposed to light.

(5) Chemical compounds including the iron (III) ion

Many exist such as FeCl3, Fe2(SO4)3, and Fe(OH)3. The intrinsic purpose is as a reagent for obtaining Iron (III) or Fe (3+) as an ion. In living organisms, it is thought as existing as heme iron in the hemoglobin of red blood cells. It should be noted that heme iron has characteristics that are different from the independent iron ion.

In particular, when acting with ascorbic acid, since it produces a hydroxyl radical (-OH) having strong oxidizing power, the iron ion is not always required when in vitro. However, in vivo, when the iron ion coexists with nitric oxide (NO), it is said that it does not always generate the hydroxyl radical (·OH).

In particular, although the iron (II) ion Fe (2+) is the reduced form of the iron (III) ion Fe (3+), there are many occasions where even with the reduced form, the oxidizing action is accentuated. In particular, if there is lipid peroxide, a radical chain reaction may easily occur. When the iron (III) ion Fe (3+) is reduced through ascorbic acid or the like, a radical generating chain reaction occurs if it coexists with lipid peroxide. In other words, it may be considered as producing many lipid radicals and having a negative effect on living organisms.

(6) Reduced ascorbic acid (chemical formula = C6H8O6)

Exists in living organisms, but it is absorbed from outside the body, and is not synthesized by humans.

(7) Glutathione (chemical formula = C10H17N3O6S)
abbreviation = GSH

Is an SH chemical compound existing in large quantities in living organisms, and it is thought that humans have a gene for synthesizing this. Glutathione is a poly-peptide configured from three amino acids (glutamic acid - cysteine - glycin = Glu-Cys-Gly), a coenzyme of glyoxylase, and is known to function as an intracellular reducing agent, an anti-aging agent, and the like. In addition, glutathione has the function of directly (nonenzymatically) reducing oxygen (O2).

(8) Cysteine (Cys)

One of the amino acids and an SH chemical compound, it is ingested as a protein and is the final product of digestive decomposition. Cysteine is a structural component of the above-mentioned glutathione and is an amino acid having an SH group. As with glutathione, two cysteines (Cys) respectively release one hydrogen atom, and become oxidized cysteine through a disulfide bond (-s-s-).

(9) Benzoic acid (chemical formula = C7H6O2)

Rarely exists in living organisms, strawberries include approximately 0.05%. Benzoic acid is a basic reducing agent and has the function of nonenzymatically and effectively scavenging the hydroxyl radical and making it into water.

(10) p-amino Benzoic acid (C7H7O2)

(11) Gallic acid (C7H6O5) (3,4,5-trihydroxy benzoic acid)

Widely exists in leaves, stems, and roots of plants, and is used as a general hemostatic agent and an antioxidant (preservative) in food (food additive). This alkaline solution has particularly strong reducing power. Gallic acid tends to react easily with oxygen.

It should be noted that those given as catalysts here are merely examples, and it is not intended to mean that they are limited to these. Accordingly, as long as contributing to the catalyzing reaction assumed by the present invention, it should be clearly noted that it does not mean that other parameters such as physical external forces including temperature, ultrasonic waves, or agitation may be excluded.

In addition, it should be added that the product of active hydrogen comprehensively includes atomic hydrogen (H·) and hydride ions (H·).

Moreover, catalysts such as those described here may be each used independently, or as needed, may be used in an appropriate mixture of a plurality of these. Basically, electrons are transmitted in the order of the hydrogen-dissolved water to catalyst to antioxidation subject, however, besides this the following orders may also be considered: the hydrogen-dissolved water to enzyme (hydrogenase) to antioxidation subject, the hydrogen- dissolved water to electron carrier to antioxidation subject, the hydrogen- dissolved water to enzyme (hydrogenase) to electron carrier to antioxidation subject, the hydrogen- dissolved water to precious metal colloid to antioxidation subject, or the hydrogen-dissolved water to precious metal colloid to electron carrier to antioxidation subject. In addition, it is possible to use such electron carrier system in combination with at least one of the electromagnetic waves selected from the group consisting of visible light, ultraviolet light, and electron beams.

Antioxidation subject

An antioxidation subject is assumed to be any subject in an oxidized state due to a deficiency in electrons or for which protection from oxidization is desired. It should be noted that oxidization mentioned here means the drawing away of electrons from a subject through the direct or indirect action of oxygen, heat, light, pH, ions, etc. In addition, to be more specific, an antioxidation subject includes for instance cells of living organisms, or subjects to be rinsed that occur in industrial fields such as industrial cleaning, food rinsing, or high precision cleaning; moreover, antioxidation substances such as vitamins, food, unregulated drugs, medical supplies, cosmetics, animal feed, oxidation/ reduction pigments (to be described later), as well as water itself, all fall within the technical scope of the present invention. It should be noted that these given as antioxidation subjects here are merely examples and it should be clearly stated here that is not intended to mean that they are limited to these.

Next, the relationship between a catalyst and an antioxidation subject is described from the standpoint of the catalyst.

(i) Hydrogen oxidation/ reduction enzyme (hydrogenase) and precious metal colloids

With the present invention, the catalyzation of the breaking reaction of the molecular hydrogen used as a substrate included in the hydrogen-dissolved water into a product of active hydrogen is performed with for example a hydrogen oxidation/ reduction enzyme, hydrogenase, or a precious metal colloid.

The reducing potential water to which a hydrogen oxidation/reduction enzyme such as the exemplary hydrogenase is added is now considered. In the case where the result of adding a low alkaline reducing potential water added with hydrogenase is ingested through drinking, and an oxidizing agent such as active oxygen species coexists with digestion-related cells (antioxidation subjects) of the living organism such as those of the intestines, this oxidizing agent is immediately reduced. In addition, when other additives such as fruit juice or a vitamin species (antioxidation subjects) coexist, the reducing potential water acts as an antioxidizing agent on these additives under the condition where hydrogenase is coexistent. Such action mechanism is considered to include the molecular hydrogen-dissolved in the reducing potential water dissociating and activating the two atomic hydrogens (H·) through the hydrogen- breaking action of the hydrogenase, the formed atomic hydrogen (H·) splitting into protons and electrons in the water, and the formed electrons then being donated to the antioxidation subject (to reduce the antioxidation subject).

The reducing potential water to which a precious metal colloid such as the exemplary platinum colloid is added is also considered. In the case where the result of adding a low alkaline reducing potential water added with Pt colloid is ingested through drinking, and an oxidizing agent such as active oxygen species coexists with digestion related cells (antioxidation subjects) of the living organism such as those of the intestines, this oxidizing agent is immediately reduced. In addition, when other additives such as fruit juice or a vitamin species (antioxidation subjects) coexist, the reducing potential water acts as the antioxidizing agent of these additives under the condition where Pt colloid is coexistent. Such action mechanism is considered to include the molecular hydrogen- dissolved in the reducing potential water dissociating and activating the two atomic hydrogens (H·) along and being adsorbed into the minute particle surface of the Pt colloid, the formed atomic hydrogen (H·) splitting into protons and electrons in the water, and the formed electrons then being donated to the antioxidation subject (to reduce the antioxidation subject).

This sort of antioxidation function is expressed only when the three items--hydrogen- dissolved water such as the reducing potential water, the hydrogen oxidation/ reduction enzyme hydrogenase or the precious metal colloid used as a catalyst, and the antioxidation subject such as the digestive system cell of the living organism--come together. In other words, the reducing power is only expressed when necessary and has no operational effect when not required. However, when looking at the chemical constitution, the reducing potential water, for instance, is nothing more than very ordinary water obtained by electrolyzing raw water. Accordingly, the fact that even after expressing reducing power, the water only acts as ordinary water and imparts no negative side effects onto, for example, the living organism is especially noteworthy. To restate this in another way, the fact that the positive effects aimed for may be obtained without the any negative effects or side effects is the critical difference from conventional antioxidation agents and active oxygen species scavenging agents.

Here, quoting the thesis of HIGUCHI, Yoshiki, an associate professor at the Faculty of Science at Kyoto University Graduate School, entitled "X-ray Structural Chemistry of Hydrogen Oxidization/ Reduction Enzymes (Suiso sanka kangen kouso no Xsen kouzou kagaku)", SPring-8 Information; Vol. 4; No. 4; July 1999, research results were announced as follows: "Hydrogen oxidization/ reduction enzymes are referred to as hydrogenase, which are proteins that are widely seen in bacteria. While generally metallic proteins containing iron, nickel or the like, recently a new hydrogenase that contains none of these metals has been discovered. Electrons occurring through the breaking of hydrogen by this molecule are used to facilitate various oxidization/ reduction reactions in the bacteria. In addition, since the proton concentration gradient at the surface layer of the cell membrane is directly governed inside and outside of the membrane, it may be thought as playing an important role in the energy/ metabolic system within the bacteria including one related to the ATP synthesis/ disassembly enzyme." In a separate thesis entitled "X-ray Crystallography of Hydrogenase Structure Through Multi-wavelength Abnormal Dispersion with Emitted Light (Hoshakou wo mochiita tahachou ijoubunsanhou niyoru hidorogenaaze no Xsen kesshou kouzou kaiseki)", the same researcher announced the following research results: "The main enzyme of the chain of reactions for an organism to obtain energy is the ATP synthesis/ disassembly enzyme. It is well known that in order to activate this enzyme, it is necessary for the proton concentration gradient to be built both inside and outside the cell membrane. The hydrogenase is a membrane protein existing in the surface layers of the cell membrane and has the function of catalyzing the oxidization/ reduction of the molecular hydrogen near the membrane. Namely, this hydrogenase directly governs the proton concentration gradient inside/ outside the membrane and controls the function of the ATP synthesis/ disassembly enzyme. Accordingly, it is likely that the hydrogenase plays an extremely important role in facilitating the energy/ metabolic system in the organism. Revealing the three-dimensional structure of the hydrogenase has significant meaning because it will unravel the relationship between the structure and function of the portion related to energy/ metabolism, the most important of the life-sustaining mechanisms."

The inventors herein, focused especially on "hydrogenase directly governs the proton concentration gradient inside/ outside the membrane and controls the function of the ATP synthesis/ disassembly enzyme. Accordingly, it is likely that the hydrogenase plays an extremely important role in facilitating the energy/ metabolic cycle in the organism." This was because the fact that the hydrogenase has such effect on the organism could be considered as proof that it (hydrogenase) may have the effect of facilitating the energy/ metabolic system due to the improved proton concentration gradient as well as expressing antioxidation function at the cell level when the antioxidation method, antioxidant- functioning water, and the living organism- applicable fluid according to the present invention are applied to living cells.

Accordingly, the hydrogen oxidation/ reduction enzyme, hydrogenase, and precious metal colloid according to the present invention can be thought of as opening the way for pharmaceuticals/ medical supplies that prevent, improve, and treat illnesses related to/ caused by monocyte/ macrophage system cellular functions, in particular, medical conditions or malfunctioning of an organ or system and illnesses related to/ caused by the increase or decrease in macrophage system cellular functions.

Specific examples of pharmaceuticals or medical products are as follows. Namely, since water generally has properties that allow it to immediately reach every location in the body including fatty membranes, cellular membranes, and the blood- brain barrier, curative effects in damaged portions may be expected by delivering hydrogen oxidation/ reduction enzyme hydrogenase or a precious metal colloid together with or separate from the hydrogen- dissolved water to the damaged portions of the living cells caused by activated oxygen through maneuvers such as an injection, intravenous drip, or dialysis.

The hydrogen oxidizing/ reducing enzyme hydrogenase here is a protein, and when assuming this is delivered to the damaged portion of the body via a maneuver such as an injection, intravenous drip, or dialysis, there is a danger that the body's immune system will recognize this as being foreign and cause an antigen antibody reaction. In order to resolve this problem, the oral tolerance principle of the body should be clinically applied. Oral tolerance refers to the antigen- specific T/B cell non- responsiveness to a foreign antigen that enters through oral/ enteral means. Simply put, oral tolerance is the phenomena where even if a substance ingested orally is a protein that may become, for example, an antigen, if it is absorbed from the small intestine, the immune tolerance allows it. Treatment using this principle has already been tested. Accordingly, through clinical application of the principle of oral tolerance, a new door of antioxidation may be opened in clinical strategy.

(ii) Visible light, ultraviolet light, and electron beams including x-rays

With the present invention, the catalyzation of the breaking reaction of the molecular hydrogen used as a substrate included in the hydrogen-dissolved water into a product of active hydrogen is performed with for example visible light, ultraviolet light or electron beams such as x-rays.
The reducing potential water on which the exemplary ultraviolet light acts as a catalyst is now considered. More specifically, in the final rinsing step in the process of performing surface treatment on a semiconductor wafer, in particular a silicon wafer, when using a reducing potential water resulting from electrolysis processing of water for electrolysis, which is deionized water to which an electrolysis auxiliary agent is added as necessary, as the silicon wafer or subject to be rinsed is rinsed while being irradiated with an ultraviolet light (wavelength ranging between approximately 150 nm and 300 nm), it is possible to reduce and protect the surface of the silicon wafer (the antioxidation subject) from oxidation as a result of releasing the seal on the reducing power intrinsic to the hydrogen through catalyzing the dissolved hydrogen in the reduced potential water with the ultraviolet light. It should be noted that when performing this rinsing, it is preferable to use reducing potential water with a pH ranging between 7 and 13. This is due to the fact that it is possible to protect the formed oxidation film on the silicon wafer surface as well as scavenge the fluorine remaining upon the silicon wafer, which is problematic from the standpoint of safety on the human body and corrosion of the device. Moreover, in the case of employing the present invention for the purposes of rinsing described herein, it is preferable that a buffered electrolysis technique using the reducing potential water generation apparatus be applied. This is because further improvement in the rinsing effect may be expected since reducing potential water with abundant dissolved hydrogen and an even lower ORP value can be obtained if generated using a buffered electrolysis technique, and expression of superior reducing power can be exhibited with the reducing potential water.
Such antioxidation function is expressed only when the three items--the hydrogen- dissolved water such as the reducing potential water, the ultraviolet light used as a catalyst, and the antioxidation subject such as the silicon wafer surface--come together. In other words, the reducing power is only exhibited when necessary and has no operational effect when not required. However, when looking at the chemical component constitution, the reducing potential water, for instance, is nothing more than very ordinary water obtained by electrolyzing raw water. Accordingly, even after demonstrating reducing power, the water only acts as ordinary water and imparts no negative effects onto, for example, the surfaces of the silicon wafer being cleaned. Moreover, since the development of silicon oxide is suppressed through this reduction, cleaning effects may be expected that are similar to the processing effects obtained through conventional multi-species acid/ alkali water mixed solution processing without causing generation of water glass, which has a possibility of becoming the cause of deterioration in electrical characteristics when formed into a device. In addition, it is possible to realize lower chemical usage levels than with conventional methods. From this standpoint, it becomes possible to secure process safety, lower usage levels of chemicals, etc., and simplify the process steps.

Antioxidant- functioning water and usage of the same

According to the present invention, an antioxidant- functioning water is provided that is characterized by adding a hydrogen oxidization/ reduction enzyme, more specifically an exemplary hydrogenase, or a precious metal colloid that catalyzes the breaking reaction of molecular hydrogen used as a substrate included in the hydrogen- dissolved water into a product of active hydrogen, to the hydrogen- dissolved water.
Of the three important factors in the present invention, since the dissolved hydrogen water and a catalyst are included in the antioxidant-functioning water employing this constitution, when put in contact with the antioxidation subject, the seal on the reducing power latently held by the hydrogen is cast off to allow expression of the antioxidation function specific to the present invention.
However, in the case where antioxidant- functioning water adopting the constitution describe above is administered through for example drinking and for instance the large intestine is the antioxidation subject, there is a problem where it is impossible to achieve the primary objective since almost all of latent reducing power of the hydrogen is unsealed before reaching the large intestine.

Therefore, it is preferable that processing or manipulation be employed on the hydrogen oxidation/ reduction enzyme, hydrogenase, or precious metal colloid used as a catalyst in order to adjust the reaction time of the catalyst.

Here, the processing or manipulation for adjusting the reaction time of the catalyst, as shown in FIG. 3, includes processing to seal the exemplary hydrogenase in an enteric capsule or the like, adjusting the pH or temperature of the hydrogenase- included antioxidant- functioning water within a range where the activation of the enzyme hydrogenase is suppressed without deactivating the activity, or the like, with the aim of having the primary catalytic action begin when the hydrogenase or a precious metal colloid reaches the subject portion such as the large intestine or small intestine. It should be noted that the optimal pH for the hydrogenase is considered to be in the neighborhood of 9, and the optimal temperature approximately 49 °C. In addition, anything that employs processing or manipulation for adjusting the reaction time of such catalyst on the hydrogenase, etc., or the environment there surrounding, falls within the technical scope of the present invention.

Meanwhile, it is essential that safety be guaranteed when using a precious metal colloid as a catalyst for application in a living organism. More specifically, it is necessary to consider biocompatibility including the acute toxicity of the precious metal colloid itself. In regards to this, with for example platinum, considering that when it is ingested by a person nearly all of it passes through the liver and is promptly eliminated in urine, and in addition, considering the fact that it has been allowed as a food additive by the Japanese Ministry of Health, Labour, and Welfare, there should be no problem with bio-compatibility. One more problem that must be considered might be the possible need to add some sort of dispersion agent in order for the precious metal colloid to disperse into the antioxidant- functioning water stably and evenly. In regards to this, for instance in the case where it will be ingested through drinking or used as a cosmetic, that which has dispersion agent function should be appropriately selected from those that have been allowed by the Japanese Ministry of Health, Labour, and Welfare as food additives. In this case, the exemplary sucrose esters of fatty acids, which are hypoallergenic and widely used in cosmetics and medical products may be favorably used.

Such antioxidant- functioning water may be considered for possible deployment in for example the following industrial fields.

Firstly, application may be made in the fields of medicine and pharmaceuticals. For example, it may be used in the manufacturing process of transfusion fluid and other medical agents. In addition, it may also be used as artificial dialysis fluid, peritoneal dialysis fluid, and pharmaceuticals. Through this, it is possible to expect prevention/treatment and secondary palliative effects on illness caused by active oxygen species.

Secondly, application may be made as a prevention/ treatment agent for aging and degeneration caused by oxidation of cutaneous tissue. For example, it may be used in the manufacturing process of cosmetic toners and other cosmetics.

Thirdly, application may be made in antioxidant food and functional food. For example, it may be considered for use in food manufacturing processes.

Fourthly, application may be made in potable water, processed water, and the like. For example, it may be considered for use as drinking water (antioxidant water), and also for use as base water in processed potable water such as canned juices, canned coffees, (PET) bottled water, and soft drinks.

Fifthly, application may be made to reduce contamination/deterioration of food due to fertilizers, herbicides, pesticides, etc., and also maintain freshness. For example, it may be used as a pre-shipment rinsing fluid for vegetables, fruits, and the like.

Sixthly, application may be made as a substitute for antiseptics, preservatives, antioxidants, and the like in prepared food manufacturing. More specifically, it may be considered for instance as a substitute for the over 347 types of food additives.

## OPERATION AND EFFECTS OF THE INVENTION

As described above, the important factors in the present invention are 1) the hydrogen- dissolved water, 2) the catalyst, and 3) the antioxidation subject. When these three factors are organically combined, the seal on the reducing power latently held by the hydrogen is cast off to allow manifest expression of the antioxidation function.

According to the antioxidation method and antioxidant- functioning water according to the present invention, an antioxidation target that is in an oxygenated state due to a deficiency of electrons, or for which oxidation protection is desired, may be transformed into a reduced state where electrons are satisfied by promoting the breaking reaction of a molecular hydrogen substrate included in the hydrogen- dissolved water into a product of active hydrogen through a process employing a catalyst on the hydrogen-dissolved water, while anticipating high benchmarks of safety on the human body and reduced environmental burden.

## BRIEF DESCRIPTION OF THE DRAWINGS

FIG. 1 is a graph showing the Nernst equation; FIG. 2 is a diagram for describing the conditions of an illumination test using an LED; FIG. 3 is a diagram for describing an exemplary application of the present invention; FIG. 4 is a schematic diagram showing a semiconductor wafer rinsing system 100 using the method of antioxidation of the present invention; FIG. 5 is a vertical cross- sectional view showing the basic configuration of a reducing potential water generation apparatus 11 used in the rinsing system 100 of the present invention; FIG. 6 and FIG. 7 are diagrams showing reduction activity evaluation test results for Pt colloid catalyst- added electrolyzed water using methylene blue color change; FIG. 8 and FIG. 9 are diagrams showing reduction activity evaluation test results for Pt colloid catalyst-added hydrogen- dissolved water using methylene blue color change; FIG. 10 and FIG. 11 are diagrams showing reduction activity evaluation test results for Pd colloid catalyst- added hydrogen- dissolved water using methylene blue color change; FIG. 12 and FIG. 13 are diagrams showing reduction activity evaluation test results for mixed precious metal (Pt + Pd) colloid catalyst- added hydrogen- dissolved water using methylene blue color change; FIG. 14 is a diagram showing reduction activity evaluation test results for Pt colloid catalyst- added electrolyzed water (pre-electrolysis processing addition vs. post-electrolysis processing addition) using methylene blue color change; FIG. 15 and FIG. 16 are diagrams showing antioxidation activity evaluation test results for Pt colloid catalyst-added electrolyzed water using DPPH radical color change; FIG. 17 and FIG. 18 are diagrams showing antioxidation activity evaluation test results for catalyst- added hydrogen- dissolved water (degasification treatment + hydrogen gas inclusion treatment) using DPPH radical color change; FIG. 19 and FIG. 20 are diagrams showing reduction activity evaluation test results for enzyme hydrogenase catalyst- added hydrogen- dissolved water (degasification treatment + hydrogen gas inclusion treatment) using methylene blue color change; FIG. 21 and FIG. 22 are diagrams for describing a method for quantitative analysis of dissolved hydrogen concentration through redox titration with oxidation/ reduction pigment; and FIG. 23 is a diagram for describing the comparison of the actually measured value and the effective value of the concentration of dissolved hydrogen DH in each type of sample water.

## BEST MODE FOR CARRYING OUT THE INVENTION

An exemplary embodiment of the present invention is described forthwith while referencing the drawings.

Referencing FIG. 4, a semiconductor wafer rinsing system 100 of this embodiment is first described. This semiconductor wafer rinsing system 100 includes a process of performing a surface treatment, for example, on a bare pattern formed by partially exposing the surface of a semiconductor wafer coated with an oxidation film using a rinsing solution such as a deionized water, a mixed solution of an acid and deionized water, or a mixed solution of an alkali and deionized water. Hydrogen- dissolved water of the present invention, in particular reducing potential water, is used for this rinsing solution. Here the antioxidation subject of the present invention is a semiconductor substrate, and ultraviolet light (described later) is used as the catalyst of the present invention.

As shown in FIG. 4, this rinsing system includes a deionized water generation apparatus 13, a reducing potential water generation apparatus 11, and a processing tank 16. The deionized water 14 produced in the deionized water generation device 13 is supplied to the inlet 111 of the reducing potential water generation apparatus 11, subjected here to electrolysis by applying a voltage to electrode plates 116 and 117, and becomes reducing potential water 15. The obtained reducing potential water 15 is then conducted into the processing tank 16 that is loaded with a semiconductor wafer W. Inside this processing tank 16, the wafer W is held with a wafer case 17, and an airtight lid 18 is provided for the processing tank 16 to prevent contamination with dust, oxygen, carbon dioxide and the like from the outside atmosphere.

In particular with this embodiment, an ultraviolet lamp 19 is provided inside this processing tank 16, and by directing ultraviolet light towards the wafer W being rinsed with the reducing potential water 15 mentioned above, catalytic action is administered to the reducing potential water.

As mentioned above, the reducing potential water obtained in the reducing potential water generation apparatus 11 of this embodiment exhibits reducing power only when necessary and does not have any operational effect when not needed. Moreover, when looking at the chemical component composition, reducing potential water, for instance, is nothing more than very ordinary water obtained by electrolyzing raw water. Accordingly, even after exhibiting reducing power, the water only acts as ordinary water and imparts no negative effects onto, for example, the surface of the silicon wafer being rinsed. Moreover, since the generation of water glass is suppressed through this reduction, rinsing effects may be expected that are similar to the processing effects obtained through conventional multi-species acid/ alkaline water mixed solution processing without causing water glass to form. In addition, it is possible to realize lower levels of chemical usage than with conventional methods. From this standpoint, it becomes possible to secure process safety, lower the amount of chemicals used, and simplify the process steps.

It should be noted that in the same drawing, reference numeral 20 denotes a hydrofluoric acid vessel, and the oxidation film on the silicon wafer may be removed by opening a valve 21 and arbitrarily adding some of the hydrofluoric acid solution in the hydrofluoric acid vessel 20 to the reducing potential water 15. In addition, reference numeral 22 in the same drawing denotes a gas/ liquid separation apparatus where unwanted gas in the reducing potential water may be removed via a valve 23.

Referencing FIG. 5, the reducing potential water generation apparatus 11 is next described in detail.

The reducing potential water generation apparatus 11 of this embodiment is formed with an inlet 111 for conducting raw water such as the deionized water, an outlet 112 for extracting the generated reducing potential water, and an electrolysis chamber 113 between the inlet 111 and the outlet 112. Although not limited to the following configuration, the reducing potential water generation apparatus 11 of this embodiment has the inlet 111 formed at the bottom of a casing 114 so as to allow conduction of raw water in a direction that is substantially perpendicular to the surface of the paper on which the drawing is shown. The outlet 112 is formed in the top portion of the casing 114 so as to allow intake of the electrolyzed water in a direction that is substantially perpendicular to the surface of the paper on which the drawing is shown.

In addition, a porous membrane 115 is provided on both the left and right inner walls of the reducing potential water generation apparatus 11, and an electrode plate 116 is provided outside each of these respective membranes 115. The other electrode plates 117 are provided inside the electrolysis chamber 113 with the respective principal surfaces thereof facing a corresponding electrode plate 116.

Thus there are two pairs of electrode plates facing each other and having a membrane sandwiched there between. These two pairs of electrode plates 116 and 117 are connected to a direct- current power source 12 that is applied with an anode attached to one of the plates in each pair of electrode plates 116 and 117, and a cathode attached to the other electrode plate. When generating reducing potential water in the electrolysis chamber 113, for example as shown in FIG. 5, the cathodes of the direct-current power source are connected to the electrode plates 117 arranged inside the electrolysis chamber 113, and the anodes are connected to the electrode plates 116 arranged outside the electrolysis chamber 113.

It should be noted that in the case of generating electrolyzed oxidation water in the electrolysis chamber 113, the anodes of the direct-current power source may be connected to the electrode plates 117 arranged inside the electrolysis chamber 113, and the cathodes may be connected to the electrode plates 116 arranged outside the electrolysis chamber 113.

It is preferable that the membrane 115 used in this embodiment have properties that allow easy permeation of water flowing through the electrolysis chamber 113 yet allow little permeated water to leak out. More specifically, with the reducing potential water generation apparatus 11 of this embodiment, during electrolysis the membrane 115 itself and the narrow space S between the membrane 115 and the electrode plate 116 forms a water screen, and electric current flows into both of the electrode plates 116 and 117 via this water screen. Accordingly, the water configuring this water screen is successively replaced, which becomes important since it increases the effectiveness of the electrolysis. In addition, if the water that permeates the membrane 115 leaks out from between the membrane 115 and the electrode plate 116, processing thereof becomes necessary, and therefore it is preferable that the membrane have water- holding properties strong enough to keep the permeated water from dripping down. However, when employing an exemplary solid electrolyte film as the membrane, since this solid electrolyte film itself has electrical conduction properties, the narrow space S formed between the membrane 115 and the electrode plate 116 may be omitted.

An exemplary membrane 115 may include a nonwoven polyester fabric or a polyethylene screen, and the film material may be a chlorinated ethylene or a polyfluorinated vinylidene and a titanium oxide or a polyvinyl chloride, and be a solid electrolyte film or a porous film having a thickness ranging between 0.1 and 0.3 mm, an average pore diameter ranging between 0.05 and 1.0 μm, and a permeable water rate that is no greater than 1.0 $cc/cm^2 \cdot min$. If a cation exchange membrane is to be utilized for the membrane 115, then a cation exchange group perfluorosufonic acid film having a base material of polytetrafluoroethylene (e.g. the Nafion(R) Membrane made by DuPont(tm)), a copolymer consisting of a cation exchange group vinyl ether and tetrafluoroethylene (e.g. flemion film made by Asahi Glass Co.), or the like may be used.

Meanwhile, the distance between the respective pairs of mutually facing electrode plates 116 and 117 sandwiching such membrane 115 may range between 0 mm and 5.0 mm, and is more preferably 1.5 mm. Here, a distance of 0 mm between the electrode plates 116 and 117 denotes the exemplary case of using a zero gap electrode wherein electrode films are formed directly on both principal surfaces of the respective membranes 115, and means that there is a distance substantially equal to the thickness of a membrane 115. It is also allowable to use zero gap electrodes where an electrode is formed on only one of the principal surfaces of a membrane 115. In addition, in the case where such a zero gap electrode is employed, it is preferable that openings or space be provided for electrode plates 116 and 117 to allow the gas that develops from the electrode surface to be released to the back surface opposite the membrane 115. It should be noted that the configuration providing such openings or space in the electrode plates 116 and 117 may also be employed for the electrode plates arranged in the electrolysis tank shown in FIG. 5.

In addition, the distance between electrode plates 117 and 117, while not specifically limited, may range between 0.5 mm and 5 mm, and more preferably is 1 mm.

In order to generate reducing potential water using the reducing potential water generation apparatus 11 with such configuration, to begin with, the negative pole (-) of the direct- current power source 12 is connected to the two electrode plates 117 and 117 arranged inside the electrolysis chamber 113, the positive pole (+) of the direct- current power source 12 is connected to the electrode plates 116 and 116 arranged outside the electrolysis chamber 113, and voltage is applied to the two pairs of mutually facing electrode plates 116 and 117 sandwiching the respective membranes 115. As the deionized water, etc., is supplied from the inlet 111, electrolysis of water is carried out in the electrolysis chamber 113, wherein the following reaction is occurring at the surface of the electrode plates 117 and in the vicinity thereof:

$$2H2\ O+2e^{-} \rightarrow 2OH- +H2 \uparrow$$

Moreover, at the surface of the electrode plates 116 outside the electrolysis chamber 113 sandwiching the membrane 115, in other words between each electrode plate 116 and membrane 115, the following reaction is occurring:

$$H2\ O-2e- \rightarrow 2H+ +1/2 \cdot O2 \uparrow$$

As this $H^+$ ion permeates the membrane 115 and passes through, a part thereof accepts an electron $e^-$ from the cathode plate 117 to become hydrogen gas dissolved in the generated electrolyzed water on the cathode side. This causes the electrolyzed water generated on the cathode side (i.e. inside the electrolysis chamber 113) to become reducing potential water having a lower oxidation/ reduction potential (ORP) than electrolyzed water generated using conventional membrane electrolysis technology.

In addition, since the remainder of the $H^+$ ion passed through the membrane 115 reacts with the $OH^-$ ion in the electrolysis chamber 113 and reverts to water, the pH of the reducing potential water generated with the electrolysis chamber 113 changes slightly towards neutrality. In other words, reducing potential water having a pH that is not very high yet having a low ORP is obtained. The reducing potential water including the hydroxide ion generated in this manner is supplied from the outlet 112.

It should be noted that when wanting to make the reducing potential water obtained through such electrolysis processing a certain desired pH level, the pH level of the raw water may be adjusted beforehand using a pH buffer acting salt solution such as phthalate, phosphate, or borate. This is because the pH of the raw water is not changed much with this reducing potential water generation apparatus 11. More specifically, for instance if a pH that tends towards alkalinity is wanted for intended applications such as rinsing silicon wafers or drinking, the pH level of the raw water may be managed and adjusted to approach alkalinity. If a pH that is substantially neutral for intended applications such as drinking, injection solution, intravenous drip solution, or dialysis fluid, the pH level of the raw water may be adjusted to be substantially neutral. Moreover, if a pH that is slightly acidic for intended applications such as cosmetics, the pH level of the raw water may be adjusted to approach slightly acidic levels.

While that shown in FIG. 5 has been described as an apparatus that generates reducing potential water in the embodiment described above, this apparatus 11 is also applicable to cases where oxidizing potential water is produced. In this case, the positive pole (+) of the direct- current power source 12 may be connected to the two electrode plates 117 and 117 arranged inside the electrolysis chamber 113, and the negative pole (-) of the direct-current power source 12 connected to the electrode plates 116 and 116 arranged outside the electrolysis chamber 113, to apply voltage to the two pairs of mutually facing electrode plates 116 and 117 sandwiching the respective membranes 115.

As the deionized water or the like is conducted from the inlet 111, electrolysis of the water is performed in the electrolysis chamber 113, wherein the following reaction is occurring at the surface of the electrode plates 117 and in the vicinity thereof:

$$H2O\ -2e^{-} \rightarrow 2H+ +1/2 \cdot O2 \uparrow$$

Meanwhile, at the surface of the electrode plates 116 outside the electrolysis chamber 113 sandwiching the membrane 115, in other words at the water screen between each electrode plate 116 and membrane 115, the following reaction is occurring:

$$2H2O\ +2e^{-} \rightarrow 2OH^{-} +H2 \uparrow$$

As this $OH^-$ ion permeates the membrane 115 and passes through, a part thereof donates an electron $e^-$ to the cathode plate 117 to become oxygen gas dissolved in the generated electrolyzed water on the anode side. This causes the electrolyzed water generated on the anode side (i.e. inside the electrolysis chamber 113) to become oxidizing potential water having a higher oxidation/ reduction potential (ORP) than electrolyzed water generated using conventional membrane electrolysis technology.

In addition, since the remainder of the $OH^-$ ion passed through the membrane 115 reacts with the $H^+$ ion in the electrolysis chamber 113 and reverts to water, the pH of the oxidizing potential water generated with the electrolysis chamber 113 changes slightly towards neutrality. In other words, oxidizing potential water having a pH that is not very low yet having a high ORP is obtained. The oxidizing potential water including the hydrogen ion generated in this manner is supplied from the outlet 112.

Incidentally, continuous water flow electrolysis processing using the reducing potential water generation apparatus 11 shown in FIG. 5 was carried out under electrolysis conditions where the cathode (-) of the direct-current power source 12 is connected to the two electrode plates 117 and 117 arranged inside the electrolysis chamber 113, the anode (+) of the direct-current power source 12 is connected to the electrode plates 116 and 116 arranged outside the electrolysis chamber 113 (electrode plate effective surface area is 1 $dm^2$), and a 5 A constant current is passed through Fujisawa City tap water having a pH of 7.9, ORP of 473 mV and flowing at a rate of 1 liter per minute. Here, a cation-exchange film made by DuPont(tm), the Nafion(R) Membrane, was used as the membrane 115, the distance between the electrode plates 116 and 117 was 1.2 mm, and the distance between electrode plates 117 and 117 inside the electrolysis chamber 113 was 1.4 mm.

As a result, a reducing potential water with a pH of 9.03 and ORP of -720 mV was obtained immediately following electrolysis processing. This reducing potential water was left to stand and the pH and ORP were measured after 5 minutes, 10 minutes, and 30 minutes. The following results were obtained: after 5 minutes, pH = 8.14 and ORP = -706 mV; after 10 minutes, pH = 8.11 and ORP = -710 mV; and after 30 minutes, pH = 8.02 and ORP = -707 mV In other words, at the time point immediately following electrolysis processing, the pH of the processing water was higher than 9 but then the pH dropped shortly thereafter, and stabilized near pH 8. This is considered as being caused by the fact that the $H^+$ ion generated near the water screen between the membrane 115 and the anode plate 116 passes through the membrane 115, moves to the electrolysis chamber 113, and then undergoes a neutralization reaction with the OH ion in this electrolysis chamber 113 to revert to the previous water. This neutralization reaction progresses with time to reach chemical equilibrium in concentration, even when the reducing potential water is left standing following electrolysis processing.

Reduction activity/ radical scavenging evaluation testing for precious metal colloid catalyst added hydrogen- dissolved water

In the following, various evaluation tests of reduction activity and radical scavenging activity as expressed through the chemical activation of inert molecular hydrogen in hydrogen- dissolved water when a precious metal colloid catalyst (platinum (Pt) colloid/ palladium (Pd) colloid) is added to the hydrogen- dissolved water of the present invention are shown through both working examples and reference examples, respectively.

In the two forms of evaluation testing mentioned above, the reduction activity evaluation testing uses methylene blue (tetramethylthionine chloride: C16H18N3ClN3S · 3(H2O)) as the antioxidation subject; on the other hand, in the radical scavenger activity evaluation testing, a radical that is relatively stable in aqueous solution, the DPPH radical (1,1-diphenyl-2-picrylhydrazyl) is used as the antioxidation subject.

Here, to describe the principle behind reduction activity evaluation for the case where methylene blue, which is categorized as an oxidation/reduction pigment, is used as the antioxidation subject, the oxidized methylene blue solution (maximum absorption wavelength of approximately 665 nm; hereafter methylene blue is also referred to as "MB") takes on a blue color, however, when this is subjected to reduction and becomes reduced methylene blue (leucomethylene blue), the color changes from the blue color to being colorless. The degree to which this blue color disappears estimates the reduction activity or in other words, the reducing power. It should be noted that while the reduced methylene blue produces a white deposit due to low solubility, as it becomes oxidized again, it becomes oxidized methylene blue and the blue color returns. That is, the color change reaction of the methylene blue solution is reversible.

Meanwhile, to describe the principle behind radical scavenging activity evaluation for the case where a DPPH radical is used as the antioxidation subject, the DPPH radical solution (maximum absorption wavelength of approximately 520 nm; hereafter may be referred to as "DPPH") takes on a deep red color, and as this DPPH is reduced and no longer a radical, this deep red color fades. The degree to which the color fades estimates the radical scavenging activity or in other words, the antioxidation power. It should be noted that the color change reaction of the DPPH radical solution is nonreversible.

The description of these evaluation tests will be made in the following order:

(1) Reducing activity evaluation of Pt colloid catalyst-added electrolyzed water using methylene blue color change

(2) Reducing activity evaluation of Pt colloid/ Pd colloid catalyst-added hydrogen- dissolved water (degasification treatment + hydrogen gas inclusion treatment) using methylene blue color change

(3) Reducing activity evaluation of Pt colloid catalyst-added electrolyzed water (pre-electrolysis processing addition/ post-electrolysis processing addition) using methylene blue color change

(4) Antioxidation activity evaluation of Pt colloid catalyst-added electrolyzed water using color change of the DPPH radical

(5) Antioxidation activity evaluation of catalyst- added hydrogen- dissolved water (degasification treatment + hydrogen gas inclusion treatment) using color change of the DPPH radical

(1) Reducing activity evaluation of Pt colloid catalyst-added electrolyzed water using methylene blue color change

(1-A): Reducing power evaluation test procedures

Standard buffer solutions 6.86 (phosphate solution) and 9.18 (borate solution) manufactured by Wako Pure Chemical Industries, Ltd. are respectively diluted to one- tenth strength in purified water to prepare pH buffer solutions. In the following, these two types of dilution water are respectively referred to as "base water 6.86" and "base water 9.18". In addition, a solution having 0.6 g of a Tanaka Kikinzoku- manufactured platinum colloid 4% solution dissolved in 500 mL of distilled water manufactured by Wako Pure Chemical Industries, Ltd. is referred to as "Pt standard solution". It should be noted that the platinum component concentration C(Pt) in the Pt standard solution becomes a 48 mg/L concentration using the formula C(Pt) = 0.6 g x 0.04 / 500 mL. Then using either base water 6.86 or base water 9.18 of the two species described above with the Pt standard solution, a total of eight species of sample solution, four species each, are prepared. These are described below:

i. base water (6.86)
ii. Pt colloid- containing solution, where 6 mL of Pt standard solution is added to 1494 mL of base water (6.86)
iii. a solution where base water (6.86) has been subjected to electrolysis processing
iv. a solution where 6 mL of Pt standard solution is added to 1494 mL of base water (6.86) to make a Pt colloid-containing solution, and this solution is subjected to electrolysis processing
v. base water (9.18)
vi. Pt colloid- containing solution, where 6 mL of Pt standard solution is added to 1494 mL of base water (9.18)
vii. a solution where base water (9.18) has been subjected to electrolysis processing
viii. a solution where 6 mL of Pt standard solution is added to 1494 mL of base water (9.18) to make a Pt colloid-containing solution, and this solution is subjected to electrolysis processing

It should be noted that the pH, ORP (mV), temperature T (°C), and Pt colloid concentration for each sample solution of the total 8 described above in i through viii are collectively shown in the following table 2.

[Table 2]

| | BASE WATER 6.86 | | | | BASE WATER 9.18 | | | |
|---|---|---|---|---|---|---|---|---|
| SAMPLE NO. | i | ii | iii | iv | v | vi | vii | viii |
| pH | 7.0 | 7.0 | 7.1 | 7.1 | 9.1 | 9.1 | 9.5 | 9.5 |
| ORP (mV) | 186 | 186 | −625 | −624 | 130 | 130 | −745 | −745 |
| Pt CONCENTRATION (μg/L) | 0 | 192 | 0 | 192 | 0 | 192 | 0 | 192 |
| TEMPERATURE (°C) | 20 | 20 | 20 | 20 | 20 | 20 | 20 | 20 |

In order to examine the respective reducing activity of each sample solution of the total 8 described above in i through viii, 10 mL of methylene blue (1 g/ L concentration) is added to 350 mL of each solution to prepare a methylene blue mole concentration of 74.4 μM, and the methylene blue light absorbance (A589: the light absorbance at wavelength 589 nm) of each sample solution is measured using a spectrophotometer.

(1-B): Disclosure of reference examples and working examples

(Reference example 1)

The methylene blue light absorbance (A589) of a solution where methylene blue is added to the catalyst- free solution (base water 6.86) of sample i is given as reference example 1, and the result thereof is shown in FIG. 6.

(Reference example 2)

The methylene blue light absorbance (A589) of a solution where methylene blue is added to the catalyst-added solution (base water 6.86 + Pt standard solution) of sample ii is given as reference example 2, and the result thereof is shown in FIG. 6.

(Reference example 3)

The methylene blue light absorbance (A589) of a solution where methylene blue is added to the catalyst- free electrolyzed water (base water 6.86 + electrolysis processing) of sample iii is given as reference example 3, and the result thereof is shown in FIG. 6.

(Working example 1)

The methylene blue light absorbance (A589) of a solution where methylene blue is added to the catalyst-added electrolyzed water (base water 6.86 + electrolysis processing + Pt standard solution) of sample iv is given as working example 1, and the result thereof is shown in FIG. 6 for comparison with reference examples 1 through 3.

(Reference example 4)

The methylene blue light absorbance (A589) of a solution where methylene blue is added to the catalyst- free solution (base water 9.18) of sample v is given as reference example 4, and the result thereof is shown in FIG. 7.

(Reference example 5)

The methylene blue light absorbance (A589) of a solution where methylene blue is added to the catalyst-added solution (base water 9.18 + Pt standard solution) of sample vi is given as reference example 5, and the result thereof is shown in FIG. 7.

(Reference example 6)

The methylene blue light absorbance (A589) of a solution where methylene blue is added to the catalyst- free electrolyzed water (base water 9.18 + electrolysis processing) of sample vii is given as reference example 6, and the result thereof is shown in FIG. 7.

(Working example 2)

The methylene blue light absorbance (A589) of a solution where methylene blue is added to the catalyst-added electrolyzed water (base water 9.18 + electrolysis processing + Pt standard solution) of sample viii is given as working example 2, and the result thereof is shown in FIG. 7 for comparison with reference examples 4 through 6.

(1-C): Examination of working examples

Examining the results of working examples 1 and 2 in comparison with those of reference examples 1 through 6, it may be said that the catalyst- added electrolyzed waters of working examples 1 and 2 has the specific methylene blue reduced irrespective of the difference in pH thereof, yet only the catalyst- added electrolyzed water exhibits significant reducing activity. It should be noted that when it was checked with the human eye whether or not there had been a change in the blue color of the methylene blue solution, only the catalyst- added electrolyzed waters of working examples 1 and 2 were colorless and clear, allowing visual confirmation that the blue color of the methylene blue had disappeared. However, visual confirmation that the blue color of the methylene blue had disappeared could not be accomplished with reference examples 1 through 6. In addition, a large amount of white- colored deposit (reduced methylene blue) was visually confirmed for the catalyst- added hydrogen- dissolved waters of working examples 1 and 2.

(2) Reducing activity evaluation of Pt colloid/ Pd colloid catalyst-added hydrogen- dissolved water (degasification treatment + hydrogen gas inclusion treatment) using methylene blue color change

(2-A): Reducing power evaluation test procedures

Solutions of Tris-HCl with a concentration of 50mM are prepared by respectively diluting a special order 1M Tris-HCl (pH 7.4) and a special order 1M Tris-HCl (pH 9.0) manufactured by Nippon Gene Co., Ltd. and sold by Wako Pure Chemical Industries, Ltd. to one- twentieth strength with distilled water manufactured by Wako Pure Chemical Industries, Ltd. In the following, these two types of dilution water are respectively referred to as "base water 7.4" and "base water 9.0". In addition, a solution having 0.6 g of a Tanaka Kikinzoku- manufactured palladium colloid 4% solution dissolved in 500 mL of distilled water manufactured by Wako Pure Chemical Industries, Ltd. is referred to as "Pd standard solution". It should be noted that the palladium component concentration C(Pd) in the Pd standard solution becomes a 48 mg/L concentration using, from the same formula as the Pt colloid, C(Pd) = 0.6 g x 0.04 / 500 mL.

Next, collecting 84 mL of base water 7.4 and base water 9.0, respectively, 4 mL of MB solution in 1 g/L concentration is added to each to prepare base water 7.4 and base water 9.0 that respectively contain a 121.7 μM concentration of methylene blue (MB). 50 mL of each of these MB-containing base waters 7.4 and 9.0 are further collected into individual degasification bottles and subjected three times to a process that includes 10 minute degasification with a vacuum pump followed by 10 minute hydrogen gas inclusion. This process aims to remove gaseous components other than hydrogen from the hydrogen- dissolved solution.

3 mL of the respective hydrogen gas- inclusioned, MB- containing base water 7.4 and base water 9.0 obtained in this manner is collected and poured into respective sealed, hydrogen gas- replaced, quartz cells. Measurements are then taken of the change in methylene blue light absorbance (ΔA572: change in light absorbance at wavelength 572 nm) that occurs when the Pt reference solution, Pd standard solution, or mixed solution of Pt standard solution and Pd standard solution with a mole ratio 1 is respectively added to the quartz cells.

(2-B): Disclosure of working examples

(Working example 3)

The change in MB light absorbance (ΔA572) in a solution where an amount of Pt standard solution sufficient to give a Pt colloid concentration of 190 μg/L has been added to MB- containing hydrogen- dissolved water (MB-containing base water 7.4 + degasification treatment + hydrogen gas inclusion treatment) is given as working example 3, and the result thereof is shown in both FIG. 8 and FIG. 9.

(Working example 4)

The change in MB light absorbance (ΔA572) in a solution where an amount of Pt standard solution sufficient to give a Pt colloid concentration of 190 μg/L has been added to MB- containing hydrogen- dissolved water (MB-containing base water 9.0 + degasification treatment + hydrogen gas inclusion treatment) is given as working example 4, and the result thereof is shown in FIG. 8 for comparison with working example 3. It should be noted that the difference between the sample waters of working example 3 and working example 4 is the pH.

(Working example 5)

The change in MB light absorbance (ΔA572) in a solution where an amount of Pt standard solution sufficient to give a Pt colloid concentration of 95 μg/L has been added to MB- containing hydrogen- dissolved water (MB-containing base water 7.4 + degasification treatment + hydrogen gas inclusion treatment) is given as working example 5, and the result thereof is shown in FIG. 9 for comparison with working example 3. It should be noted that the difference between the sample waters of working example 3 and working example 5 is the Pt colloid concentration.

(Working example 6)

The change in MB light absorbance (ΔA572) in a solution where an amount of Pd standard solution sufficient to give a palladium colloid concentration of 444 μg/L has been added to MB- containing hydrogen dissolved water (MB-containing base water 7.4 + degasification treatment + hydrogen gas inclusion treatment) is given as working example 6, and the result thereof is shown in both FIG. 10 and FIG. 11.

(Working example 7)

The change in MB light absorbance (ΔA572) in a solution where an amount of Pd standard solution sufficient to give a palladium colloid concentration of 444 μg/L has been added to MB- containing hydrogen dissolved water (MB-containing base water 9.0 + degasification treatment + hydrogen gas inclusion treatment) is given as working example 7, and the result thereof is shown in FIG. 10 for comparison with working example 6. It should be noted that the difference between the sample waters of working example 6 and working example 7 is the pH.

(Working example 8)

The change in MB light absorbance (ΔA572) in a solution where an amount of Pd standard solution sufficient to give a palladium colloid concentration of 111 μg/L has been added to MB- containing hydrogen dissolved water (MB-containing base water 7.4 + degasification treatment + hydrogen gas inclusion treatment) is given as working example 8, and the result thereof is shown in FIG. 11 for comparison with working example 6. It should be noted that the difference between the sample waters of working example 6 and working example 8 is the palladium colloid concentration.

(Working example 9)

The change in MB light absorbance (ΔA572) in a solution where an amount of a mixed solution of Pt standard solution and Pd standard solution with a mole ratio of 1 sufficient to give a precious metal mixed (Pt + Pd) colloid concentration of 160 μg/L has been added to MB- containing hydrogen- dissolved water (MB- containing base water 7.4 + degasification treatment + hydrogen gas inclusion treatment) is given as working example 9, and the result thereof is shown in both FIG. 12 and FIG. 13.

(Working example 10)

The change in MB light absorbance (ΔA572) in a solution where an amount of mixed solution, similar to working example 9, sufficient to give a precious metal mixed (Pt + Pd) colloid concentration of 160 μg/L has been added to MB- containing hydrogen- dissolved water (MB- containing base water 9.0 + degasification treatment + hydrogen gas inclusion treatment) is given as working example 10, and the result thereof is shown in FIG. 12 for comparison with working example 9. It should be noted that the difference between the sample waters of working example 9 and working example 10 is the pH.

(Working example 11)

The change in MB light absorbance (ΔA572) in a solution where an amount of mixed solution, similar to working example 9, sufficient to give a precious metal mixed (Pt + Pd) colloid concentration of 80 μg/L has been added to MB- containing hydrogen- dissolved water (MB- containing base water 7.4 + degasification treatment + hydrogen gas inclusion treatment) is given as working example 11, and the result thereof is shown in FIG. 13 for comparison with working example 9. It should be noted that the difference between the sample waters of working example 9 and working example 11 is the precious metal (Pt + Pd) colloid concentration.

(2-C): Examination of working examples

FIG. 8, which compares working examples 3 and 4, shows the MB reducing activity of Pt colloid- added hydrogen- dissolved water occurring at pH 7.4 and pH 9.0. According to this diagram, both examples show high levels of MB reducing activity without seeing a substantial difference in MB reducing activity due to difference in pH.

FIG. 9, which compares working examples 3 and 5, shows the MB reducing activity of Pt colloid- added hydrogen- dissolved water occurring at Pt colloid concentrations of 95 μg/L and 190 μg/L. According to this diagram, the higher Pt colloid concentration also has higher MB reducing activity. From this, an increase in Pt colloid concentration may be considered effective towards increasing MB reducing activity.

FIG. 10, which compares working examples 6 and 7, shows the MB reducing activity of Pd colloid- added hydrogen- dissolved water occurring at pH 7.4 and pH 9.0. According to this diagram, both examples show high levels of MB reducing activity without seeing a substantial difference in MB reducing activity due to difference in pH.

FIG. 11, which compares working examples 6 and 8, shows the MB reducing activity of Pd colloid- added hydrogen- dissolved water occurring at Pd colloid concentrations of 111 μg/L and 444 μg/L. According to this diagram, the higher Pd colloid concentration also has higher MB reducing activity. From this, an increase in Pd colloid concentration may be considered effective towards increasing MB reducing activity.

FIG. 12, which compares working examples 9 and 10, shows the MB reducing activity of precious metal mixed (Pt + Pd) colloid- added hydrogen-dissolved water occurring at pH 7.4 and pH 9.0. According to this diagram, both examples show high levels of MB reducing activity without seeing a substantial difference in MB reducing activity due to difference in pH.

FIG. 13, which compares working examples 9 and 11, shows the MB reducing activity of precious metal mixed (Pt + Pd) colloid- added hydrogen-dissolved water occurring at precious metal mixed (Pt + Pd) colloid concentrations of 80 μg/L and 160 μg/L. According to this diagram, the higher precious metal mixed (Pt + Pd) colloid concentration also has higher MB reducing activity. From this, an increase in precious metal mixed (Pt + Pd) colloid concentration may be considered effective towards increasing MB reducing activity.

In addition, comparing FIG. 8 (working examples 3 and 4: MB reducing activity of Pt colloid- added hydrogen-dissolved water) and FIG. 10 (working examples 6 and 7: MB reducing activity of Pd colloid- added hydrogen- dissolved water), it may be understood that although working examples 3 and 4 have lower concentrations, these show substantially the same MB reducing activity as working examples 6 and 7. Moreover, comparing the mole concentrations (μM) of both, since the Pt colloid is 0.98 μM and the Pd colloid 4.17 μM, the Pt colloid uses a lower mole concentration. This means that regarding MB reducing activity expected for the precious metal catalyst according to the present invention, it may be said that the Pt colloid is superior to the Pd colloid because substantially the same MB reducing activity can be obtained with a smaller dosage.

Meanwhile, comparing FIG. 8 (working examples 3 and 4: MB reducing activity of Pt colloid- added hydrogen-dissolved water) and FIG. 12 (working examples 9 and 10: MB reducing activity of precious metal mixed (Pt + Pd) colloid- added hydrogen- dissolved water), it may be understood that both show superior MB reducing activity. Even comparing the mole concentrations (μM) of both, since the Pt colloid is 0.98 μM and the precious metal mixed (Pt + Pd) colloid 1.07 μM, both are substantially the same. Therefore, regarding MB reducing activity expected for the precious metal catalyst according to the present invention, the Pt colloid and the precious metal mixed (Pt + Pd) colloid are substantially the same.

(3) Reducing activity evaluation of Pt colloid catalyst-added electrolyzed water (pre- electrolysis processing addition/ post- electrolysis processing addition) using methylene blue color change

(3-A): Reducing power evaluation test procedures

2000 mL of base water 6.86 similar to that prepared in (1-A) described above is prepared, and 4 mL of Pt standard solution from this is added to 1000 mL to prepare approximately 1 liter of Pt colloid- containing base water 6.86. For the time being, the Pt colloid is not added to the remaining 1000 mL. In this manner, approximately 1 liter of Pt colloid- free base water 6.86 and approximately 1 liter of Pt colloid- containing base water 6.86 are prepared.

Next, both of the samples are subjected to electrolysis processing separately. 2.86 mL of the respective obtained electrolyzed waters (hydrogen- dissolved water) is collected and poured into respective sealed, hydrogen gas-replaced quartz cells.

Moreover, only 0.14 mL of the 1 g/L concentration MB solution that has been degasified and hydrogen gas inclusioned beforehand is added to the Pt colloid- free cell. At this point, both cells are set in the spectrophotometer and placed on stand-by.

Next, 12 μL in a 48 mg/L concentration of Pt colloid solution is added to the Pt colloid- free cell, and into the Pt colloid- containing cell, 0.14 mL of 1 g/L concentration MB solution that has already been through degasification treatment and hydrogen gas inclusion treatment is added, and measurement of both cell solutions is begun. It should be noted that the Pt colloid concentrations added to each cell are prepared so that each respectively becomes approximately 182 μg/L.

(3-B): Disclosure of working examples

(Working example 12)

The minimum value of MB light absorbance (A572: the light absorbance at wavelength 572 nm) of the pre-catalyst addition electrolyzed water (MB- containing base water 6.86 + Pt colloid pre-electrolysis addition) that occurs within 30 minutes from the start of measurement is given as working example 12, and the result thereof is shown in FIG. 14.

(Working example 13)

The minimum value of MB light absorbance (A572) of the post-catalyst addition electrolyzed water (MB- containing base water 6.86 + Pt colloid post-electrolysis addition) that occurs within 30 minutes from the start of measurement is given as working example 13, and the result thereof is shown in FIG. 14 for comparison with working example 12.

(3-C): Examination of working examples

FIG. 14, which compares working examples 12 and 13, shows the MB reducing activity of electrolyzed water when the period of adding the Pt colloid is different (before vs. after electrolysis processing). According to this diagram, it may be understood that adding the Pt colloid before electrolysis processing allows higher MB reducing activity to be obtained. The reason for this is still being studied, however it is speculated that this stems from the activated hydrogen at the root of the MB reducing activity making the oxidizing power of the oxidant such as oxygen in the electrolyzed water ineffective. This is the reason derived from the fact that when the dissolved oxygen concentration of the electrolyzed water on which electrolysis processing had been implemented using Pt colloid -containing activated carbon processing water as the raw water was measured immediately after electrolysis processing thereof, the concentration of dissolved oxygen in this electrolyzed water was found to be substantially zero. Should this be the case, not only in this exemplary electrolysis processing, but also in hydrogen inclusion treatment or hydrogen gas bubbling processing, pre-processing addition of the catalyst (Pt colloid) is considered preferable from the standpoint that higher levels of MB reducing activity are obtained (because of the oxidizing power of the oxidant such as oxygen being made ineffective). Moreover, even in the case of obtaining dissolved hydrogen water by employing processing where, for instance, a reducing agent is added to the raw water, addition of the Pt colloid to the raw water beforehand is considered preferable from the standpoint that higher levels of MB reducing activity similar to that described above may be obtained. It should be noted that the catalyst is not limited to the Pt colloid. Pre-processing addition of a catalyst such as Pd colloid, or mixed colloid of Pt colloid and Pd colloid is similarly preferable from the standpoint of obtaining higher levels of MB reducing activity.

(4) Antioxidation activity evaluation of Pt colloid catalyst-added electrolyzed water using color change of the DPPH radical

(4-A): Antioxidation activity evaluation test procedures

In order to examine the respective antioxidation activity of each sample solution of the total 8 samples i through viii shown in table 2, similar to that prepared in (1-A) above, 4 mL of DPPH (0.16 g/L concentration) is added to 16 mL of each solution to prepare a DPPH mole concentration of 81.15 (μM), and the change in DPPH light absorbance (A540: the light absorbance at wavelength 540 nm) of each solution 3 minutes after adding the DPPH is measured using a spectrophotometer.

(4-B): Disclosure of reference examples and working examples

(Reference example 7)

The change in DPPH light absorbance (ΔA540) of a solution where DPPH is added to the catalyst- free solution (base water 6.86) of sample i is given as reference example 7, and the result thereof is shown in FIG. 15. It should be noted that the change in DPPH light absorbance (ΔA540) in the same drawing shows the difference (ΔA540) between the light absorbance of this sample i (blank) and the light absorbance of samples i through iv. Accordingly, the change in DPPH light absorbance (ΔA540) for reference example 7 is zero.

(Reference example 8)

The change in DPPH light absorbance (ΔA540) of a solution where DPPH is added to the catalyst- added solution (base water 6.86 + Pt standard solution) of sample ii is given as reference example 8, and the result thereof is shown in FIG. 15.

(Reference example 9)

The change in DPPH light absorbance (ΔA540) of a solution where DPPH is added to the catalyst- free solution (base water 6.86 + electrolysis processing) of sample iii is given as reference example 9, and the result thereof is shown in FIG. 15.

(Working example 14)

The change in DPPH light absorbance (ΔA540) of a solution where DPPH is added to the catalyst- added electrolyzed water (base water 6.86 + electrolysis processing + Pt standard solution) of sample iv is given as working example 14, and the result thereof is shown in FIG. 15 for comparison with reference examples 7 through 9.

(Reference example 10)

The change in DPPH light absorbance (ΔA540) of a solution where DPPH is added to the catalyst- free solution (base water 9.18) of sample v is given as reference example 10, and the result thereof is shown in FIG. 16. It should be noted that the change in DPPH light absorbance (ΔA540) in the same drawing shows the difference (ΔA540) between the light absorbance of this sample v (blank) and the light absorbance of samples v through viii. Accordingly, the change in DPPH light absorbance (ΔA540) for reference example 10 is zero.

(Reference example 11)

The change in DPPH light absorbance (ΔA540) of a solution where DPPH is added to the catalyst- added solution (base water 9.18 + Pt standard solution) of sample vi is given as reference example 11, and the result thereof is shown in FIG. 16.

(Reference example 12)

The change in DPPH light absorbance (ΔA540) of a solution where DPPH is added to the catalyst- free electrolyzed water (base water 9.18 + electrolysis processing) of sample vii is given as reference example 12, and the result thereof is shown in FIG. 16.

(Working example 15)

The change in DPPH light absorbance (ΔA540) of a solution where DPPH is added to the catalyst- added electrolyzed water (base water 9.18 + electrolysis processing + Pt standard solution) of sample viii is given as working example 15, and the result thereof is shown in FIG. 16 for comparison with reference examples 10 through 12.

(4-C): Examination of working examples

Examining the results of working examples 14 and 15 in comparison with those of reference examples 7 through 12, it may be said that the catalyst- added electrolyzed waters of working examples 14 and 15 has the specific DPPH radical scavenged with both base waters 6.86 and 9.18, and shows significant antioxidation activity and radical scavenging activity. However, the Pt colloid catalyst was added before electrolysis processing. It should be noted that, as shown in FIG. 15, DPPH radical scavenging activity is found in reference example 9 even though catalyst- free electrolyzed water is used. This may be considered as suggesting possible expectation of the expression of antioxidation activity in electrolyzed water having a high concentration of dissolved hydrogen through the pH conditions, etc. thereof, even without the assistance of a catalyst.

(5) Antioxidation activity evaluation of catalyst-added hydrogen dissolved water (degasification treatment + hydrogen gas inclusion treatment) using color change of the DPPH radical

(5-A): Antioxidation activity evaluation test procedures

"Base water 7.4" and "base water 9.0" are prepared as with that prepared in (2-A) above. Next, 406 μM of DPPH solution and 50 mL each of base water 7.4 and base water 9.0 are collected and subjected three times to a process that includes 10 minute degasification with a vacuum pump followed by 10 minutes of hydrogen gas infusion. This process aims to remove gaseous components other than hydrogen from the hydrogen-dissolved water.

0.3 mL of the hydrogen gas- inclusioned DPPH solution obtained in this manner, and 2.7 mL each of base water 7.4 and base water 9.0 are collected and poured into respective sealed, hydrogen gas- replaced, quartz cells. Measurements of the change in DPPH light absorbance (ΔA540: change in light absorbance at wavelength 540 nm) for both that to which the Pt standard solution has been added and that to which it has not are then taken over 30 minutes respectively using a spectrophotometer.

(5-B): Disclosure of reference examples and working examples

(Reference example 13)

The change in DPPH light absorbance (ΔA540) of a solution where Pt standard solution has not been added to the hydrogen- dissolved water (base water 7.4 + degasification treatment + hydrogen gas inclusion treatment) is given as reference example 13, and the result thereof is shown in FIG. 17.

(Working example 16)

The change in DPPH light absorbance (ΔA540) in a solution where an amount of Pt standard solution sufficient to give a Pt colloid concentration of 190 μg/L has been added to hydrogen- dissolved water (base water 7.4 + degasification treatment + hydrogen gas inclusion treatment) is given as working example 16, and the result thereof is shown in FIG. 17 for comparison with reference example 13. It should be noted that the difference between reference example 13 and working example 16 is whether or not the Pt colloid has been added.

(Reference example 14)

The change in DPPH light absorbance (ΔA540) of a solution where Pt standard solution has not been added to the hydrogen- dissolved water (base water 9.0 + degasification treatment + hydrogen gas inclusion treatment) is given as reference example 14, and the result thereof is shown in FIG. 18.

(Working example 17)

The change in DPPH light absorbance (ΔA540) in a solution where an amount of Pt standard solution sufficient to give a Pt colloid concentration of 190 μg/L has been added to hydrogen- dissolved water (base water 9.0 + degasification treatment + hydrogen gas inclusion treatment) is given as working example 17, and the result thereof is shown in FIG. 18 for comparison with reference example 14. It should be noted that the difference between reference example 14 and working example 17 is whether or not the Pt colloid has been added.

(5-C): Examination of working examples

FIG. 17, which compares reference example 13 and working example 16, shows the DPPH radical scavenging activity in pH 7.4 hydrogen-dissolved water where the difference is whether or not the Pt colloid is added. Similarly, FIG. 18, which compares reference example 14 and working example 17, shows the DPPH radical scavenging activity in pH 9.0 hydrogen- dissolved water where the difference is whether or not the Pt colloid is added. According to these diagrams, with the Pt colloid- free reference examples 13 and 14, the change in light absorbance seen may be considered as only that corresponding to natural fading during the duration of measurement (30 minutes). Meanwhile, with the Pt colloid- containing working examples 16 and 17, the expression of DPPH radical scavenging that clearly surpasses natural fading is observed. It should be noted that there was no substantial difference observed in levels of DPPH radical scavenging due to difference in pH.

Reducing activity evaluation testing of enzyme hydrogenase catalyst- added hydrogen- dissolved water

Next, evaluation of reduction activity as expressed through the chemical activation of inert molecular hydrogen in hydrogen- dissolved water when an enzyme hydrogenase catalyst is added to the hydrogen- dissolved water of the present invention is shown respectively through both working examples and reference examples, respectively. In this reduction activity evaluation test, the oxidization/reduction pigment methylene blue is used as the antioxidation subject as with the reduction activity testing for precious metal colloid catalyst- added hydrogen- dissolved water. Since the reducing activity evaluation principle in this case is similar to that described for the precious metal colloid catalyst above, repetitive description thereof is omitted.

(6) Reducing activity evaluation of enzyme hydrogenase catalyst-added hydrogen- dissolved water (degasification treatment + hydrogen gas inclusion treatment) using methylene blue color change

(6-A): Reducing activity evaluation test procedures

In the same manner as that prepared in (2-A) above, "base water 7.4" and "base water 9.0" are prepared. Next, collecting 84 mL of each of base water 7.4 and base water 9.0, respectively, 4 mL of MB solution in 1 g/L concentration is added to each to prepare base water 7.4 and base water 9.0 that respectively contain a 121.7 μM concentration of methylene blue (MB). 50 mL of each of these MB- containing base waters 7.4 and 9.0 are further collected and subjected three times to a process that includes 10 minute degasification with a vacuum pump followed by 10 minute hydrogen gas inclusion. This process aims to remove gaseous components other than hydrogen from the hydrogen- dissolved water. Meanwhile, a 125 μM concentration of hydrogenase solution is diluted with distilled water to one fourth strength. This is then poured into 1 mL microcapsules and the oxygen is removed by infusing these capsules with nitrogen gas (inert gas).

3 mL of the respective hydrogen gas- inclusioned, MB- containing base water 7.4 and base water 9.0 obtained in this manner is collected and poured into respective sealed, hydrogen gas- replaced, quartz cells. Measurements are then taken of the change in methylene blue light absorbance (ΔA572) that occurs when the hydrogenase solution prepared as described above is added to the quartz cells.

(6-B): Disclosure of reference examples and working examples

(Working example 18)

The change in MB light absorbance (ΔA572) in a solution where 10 μL of the hydrogenase solution prepared as described above has been added to MB- containing hydrogen- dissolved water (MB- containing base water 7.4 + degasification treatment + hydrogen gas inclusion treatment) is given as working example 18, and the result thereof is shown in FIG. 19.

(Reference example 15)

The change in MB light absorbance (ΔA572) in a solution where the hydrogenase solution has not been added to MB- containing hydrogen-dissolved water (MB- containing base water 7.4 + degasification treatment + hydrogen gas inclusion treatment) is given as reference example 15, and the result thereof is shown in FIG. 19 for comparison with working example 18. It should be noted that the difference between the sample waters of working example 18 and reference example 15 is whether or not the enzyme hydrogenase has been added.

(Working example 19)

The change in MB light absorbance (ΔA572) in a solution where 10 μL of the hydrogenase solution prepared as described above has been added to MB- containing hydrogen- dissolved water (MB- containing base water 9.0 + degasification treatment + hydrogen gas inclusion treatment) is given as working example 19, and the result thereof is shown in FIG. 20.

(Reference example 16)

The change in MB light absorbance (ΔA572) in a solution where the hydrogenase solution has not been added to MB- containing hydrogen-dissolved water (MB- containing base water 9.0 + degasification treatment + hydrogen gas inclusion treatment) is given as reference example 16, and the result thereof is shown in FIG. 20 for comparison with working example 19. It should be noted that the difference between the sample waters of working example 19 and reference example 16 is whether or not the enzyme hydrogenase has been added.

(6-C): Examination of working examples

Examining the results of working examples 18 and 19 in comparison with those of reference examples 15 and 16, it may be said that the catalyst-added hydrogen- dissolved waters of working examples 18 and 19 have the methylene blue specifically reduced irrespective of the difference in pH thereof, yet only the catalyst- added hydrogen- dissolved water exhibits significant reducing activity. It should be noted that when it was checked with the human eye whether or not there had been a change in the blue color of the methylene blue solution, only the catalyst- added hydrogen- dissolved waters of working examples 18 and 19 were colorless and clear, allowing visual confirmation that the blue color of the methylene blue had disappeared. However, visual confirmation that the blue color of the methylene blue had disappeared could not be accomplished with reference examples 15 and 16. In addition, a large amount of white- colored deposit (reduced methylene blue) was visually confirmed for the catalyst- added hydrogen- dissolved waters of working examples 18 and 19. Quantitative analysis of dissolved hydrogen concentration through oxidation/ reduction titration of oxidation/ reduction pigment

(A) Development of idea

It has been proven that hydrogen generated through the negative reaction during electrolysis processing is dissolved in the electrolyzed water (electrolyzed reducing water) that has been subjected to electrolysis processing in the reducing potential water generation apparatus 11 developed by the applicants. Approximately what concentration of hydrogen is dissolved in this electrolyzed water may be measured in a way with a dissolved hydrogen meter. Here, the expression "in a way" is used because generally used dissolved hydrogen meters employ a measuring principle whereby electrochemical physical quantities occurring in the electrode reaction are replaced with the concentration of dissolved hydrogen using a table look-up protocol so that the readings tend to vary significantly depending on the external causes such as liquid properties of the test water.

However, as description was made based on the working examples already described above, with the catalyst-free electrolyzed water where no catalyst is added to the electrolyzed water, even when an oxidation/reduction pigment (an antioxidation subject) such as oxidized methylene blue is added, this pigment does not show the color change specific to the reduction reaction; but on the other hand, with catalyst- added electrolyzed water where a catalyst has been added to the electrolyzed water, when this pigment is added, the pigment shows the color change specific to the reduction reaction. In other words, the oxidation/ reduction reaction of the oxidation/ reduction pigment may be visually recognized by observing the change in color of the solution (catalyst- added electrolyzed water + oxidation/ reduction pigment).

Through a process of trial and error as this testing was repeated, the inventors realized that the color change reaction of the oxidation/ reduction pigment methylene blue from blue to clear tended to occur more swiftly as the reducing power of the catalyst- added electrolyzed water increased. More specifically, when comparing the reducing power of the catalyst- added electrolyzed water and the reducing power consumed to reduce the oxidation/reduction pigment methylene blue that is added, some sort of correlation was noticed between the size of the residual reducing power or the difference between the two reducing powers when the former is larger than the latter, and the speed of the color change reaction of the oxidation/ reduction pigment methylene blue.

In keeping with this discovery, as zealous research on the possible industrial utilization of this correlation progressed, the inventors ended up wondering if it was possible to perform quantitative analysis of the explicit antioxidation power (dissolved hydrogen concentration) of the catalyst-added electrolyzed water through the oxidation/ reduction reaction of the oxidation/ reduction pigment methylene blue .

(B) Testing objectives

When a solution with a predetermined concentration of oxidation/reduction pigment methylene blue is dripped into the hydrogen- dissolved water that includes catalyst- added electrolyzed water, the fact that the total dripped amount of methylene blue added until this post-drip solution no longer causes the reducing color reaction to be displayed (hereafter, also referred to as "equivalence point") becomes a measure of the quantitative analysis of the dissolved hydrogen concentration (explicit antioxidation power) is verified through the following tests.

(C) Outline of effective dissolved hydrogen concentration quantitative analysis method

In order to quantitatively analyze the effective amount of reducing power (antioxidation power) expressed through the chemical activation of inert molecular hydrogen in the hydrogen- dissolved water, or in other words, the effective dissolved hydrogen concentration DH (mg/L) when a catalyst is added to the hydrogen- dissolved water according to the present invention, methylene blue oxidation/ reduction titration was carried out on the catalyst-(Pt colloid) added hydrogen- dissolved water using Pt colloid as the catalyst and methylene blue as the oxidation/ reduction pigment.

(D) Testing procedures

The basic testing procedures include preparing a number of sample waters (already having respective features such as dissolved hydrogen concentration measured), adding the catalyst (Pt colloid) to these samples, and delivering drops of the methylene blue. Comparative evaluation is then made of whether or not there exists correlation between the effective amount of dissolved hydrogen concentration found from each total amount of methylene blue added and the actual reading of the dissolved hydrogen meter.

If there is a correlation between the two, it can be considered that the legitimacy of the dissolved hydrogen concentration quantitative analysis through methylene blue redox titration, and the fact that the key material expressing the explicit antioxidant function is dissolved hydrogen can be objectively validated.

In keeping with such basic thinking, to begin with, a one-fortieth strength Pt standard solution is prepared by diluting the Pt standard solution described earlier to a concentration of one-fortieth strength. It should be noted that the platinum component concentration C(Pt) in the one-fortieth strength Pt standard solution becomes a 192 mg/L concentration using the formula C(Pt) = 24 g x 0.04 / 500 mL.

Next, a 1 g/L concentration (mole concentration by volume: 2677.4 μM) of methylene blue solution and a 10 g/L concentration (mole concentration by volume: 26773.8 μM) of methylene blue solution are prepared. Here, two types of different concentrations of methylene blue solution are prepared because changing the concentration of the methylene blue solution to be added in response to the hydrogen concentration which would be dissolved in the water to be tested is expected to result in allowing the added amount of the solution to be reduced and improve test accuracy. Nevertheless, the Pt concentration in the Pt standard solution and the MB concentration in the methylene blue solution are not limited to these, but may be adjusted as appropriate in response to conditions such as the amount of hydrogen which would be dissolved in the water to be tested.

Next, 50 mL of one-fortieth strength Pt standard solution prepared as described above and 50 mL of each of the two types of different concentrations of methylene blue solution are respectively collected in individual degasification bottles, these are subjected three times to a process that includes 10 minutes of degasification using a vacuum pump followed by 10 minutes of nitrogen gas inclusion, and the methylene blue solution and one-fortieth strength Pt standard solution that has undergone the nitrogen gas replacement. This process aims to remove other gaseous components besides nitrogen (inert gas) in each of the solutions.

Next, 200 mL of test water is poured into an acrylic, gas-impermeable tester together with a magnet stirrer. This tester has been created for this testing and has a structure whereby the bottom is formed by attaching a round acrylic plate to one end along the length of a hollow, cylinder-shaped, acrylic tube, and the open end has a structure that has a pusher configured with a round plate having a diameter that is slightly smaller than the inner diameter of this tube so as to seal in a piston-like manner allowing movement along the length of the tube. On the inside wall of this tester, a solution injection part configured with a hollow, cylinder-shaped, acrylic tube directed so as to radiate out towards the outside wall is provided in this tester to allow injection of MB solution or one-fortieth strength Pt standard solution separated from the outside environment into the test water holding compartment demarcated by the bottom surface, side wall, and pusher of this tester. In addition, a removable rubber stopper is provided for this solution injection part to allow syringe needle insertion. When pouring the test water into the test water holding compartment of the tester configured in this manner, the test water is softly pumped while the pusher is removed from the tester and then the pusher is attached to prevent vapor from forming inside the test water holding compartment. This allows the test water inside the test water holding compartment of the tester to be sealed in a condition separate from the outside environment. In addition, when the one-fortieth strength Pt standard solution or MB solution is poured into the test water holding compartment of the tester, such solution is collected through suction to prevent vapor from developing inside the syringe. The solution is softly injected by inserting the needle of the syringe into the rubber stopper equipped with a solution injection part and pushing the piston of the syringe. It should be noted that the tester disclosed here is merely an example. Other appropriate vessels may be used as long as they meet conditions including:

gas-impermeable material;
test water holding compartment can be isolated from outside environment;
volume of test water holding compartment is adjustable;
test water holding compartment is air-tight and water-tight;
one-fortieth strength Pt standard solution and MB solution may be poured in while the test water holding compartment is isolated from the outside environment; and
the stirrer is moveable.

Next, the tester containing the test water described above is placed bottom- down on a magnetic stirring table and stirring with the stirrer is begun.

Next, 1 mL of the one-fortieth strength Pt standard solution that has been subjected to the nitrogen gas replacement described above is injected to the test water holding compartment using a syringe and this is sufficiently stirred and mixed.

Next, a predetermined density of methylene blue solution that has undergone the above- mentioned nitrogen gas replacement is injected a little bit at a time using a syringe while visually observing the color change of the test water. Here, if the dissolved hydrogen concentration of the test water is greater than the amount of methylene blue poured in, then the methylene blue is reduced and becomes colorless. However, as the amount of methylene blue solution poured in gradually increases, the added methylene blue and the dissolved hydrogen of the test water counteract each other, and in time the change in the methylene blue from blue to colorless can no longer be observed. Making this point the equivalence point, the concentration of dissolved hydrogen DH in the test water can be found from the methylene blue concentration of the methylene blue solution and the total amount of methylene blue solution added.

(E) Finding the effective concentration of dissolved hydrogen

In the following, the meaning of the "effective dissolved hydrogen concentration DH" is explained while showing the formula for finding the effective dissolved hydrogen concentration DH in the test water from the concentration and total added amount of the methylene blue solution added to the test water and the process of deriving the formula.
To begin with, in the following description, the volume of water to be tested is given as 200 mL and the methylene blue volume mole concentration of the methylene blue solution to be added to the test water is given as N (μmol/L).
Moreover, given that the total amount of methylene blue solution added to reach the equivalence point is A (mL), the total added amount of methylene molecules B(mol) becomes

$$B = N \cdot A(\mu mol / L \times mL)$$

$$= N \cdot A(m\ \mu mol) \qquad \text{(Equation 1)}$$

Here, given that the chemical formula of the methylene blue molecule is given as MBCl, and the chemical formula of the hydrogen molecule as $H_2$, the reaction in the solution between the hydrogen molecule activated by the Pt colloid and the methylene blue molecule may be expressed with the following reaction formula 1.

$$H_2 + MBCl \rightarrow HCl + MBH \qquad \text{(Reaction formula 1)}$$

Here, HCl is hydrochloric acid, and MBH is reduced methylene blue. According to reaction formula 1, 1 mole of hydrogen molecules and 1 mole of methylene blue molecules react and generate 1 mole of reduced methylene blue molecules. In order to explain the reception of electrons, the reaction formula may be written divided into two half equations as follows:

$$H2 \rightarrow H+ + (H+ + 2e^-) \qquad \text{(Half equation 1)}$$

$$MB+ + (H+ + 2e^-) \rightarrow MBH \qquad \text{(Half equation 2)}$$

Half reaction 1 means that the 1 mole of hydrogen molecules releases 2 mole of electrons, and half equation 2 means that the 1 mole of methylene blue cations, or 1 mole of methylene blue molecules accepts 2 mole of electrons. Here, 1 mole of hydrogen molecules is equivalent to 2 g since 2 mole of electrons are released. Meanwhile, 1 mole of methylene blue cations, or 1 mole of methylene blue molecules is equivalent to 2 g since 2 mole of electrons are accepted. As a result, since the gram equivalence of both the hydrogen molecule and the methylene blue cation, or the methylene blue molecule is 2, the hydrogen molecule and the methylene blue molecule react at a rate of 1 to 1 in terms of the mole ratio.
In keeping with this, the total amount of methylene blue B added to the test water described above is also the amount of hydrogen molecules consumed.
Accordingly, given a total amount of hydrogen molecules to be measured as C(mμmol), the following may be obtained from Equation 1:

$$C = B = N \cdot A(m\ \mu mol) \qquad \text{(Equation 2)}$$

Moreover, if the volume of test water is 200 mL and the value of the effective hydrogen molecule mole concentration by volume $H_2$ (mol/L) of the test water is the mole count C(mol) divided by volume (mL), then

$$H_2 \text{ (mol/L)} = C / 200 (\text{m } \mu\text{mol/mL})$$

$$= C / 200 (\mu\text{mol/L})$$

Moreover, in the case of exchanging this unit with mass concentration (g/L), given the corresponding mass concentration of hydrogen molecules as D, from the proportional expression relating to the hydrogen molecule $H_2$:

$$1 \text{ mole} / 2 \text{ g} = H_2 (\mu\text{mol/L}) / D \qquad \text{(Equation 4)}$$

if this Equation 4 is replaced with Equation 3, then

$$D = 2 \cdot C/200 (\mu\text{g/L})$$

$$= C /100 (\mu\text{g/L}) \qquad \text{(Equation 5)}$$

This is the mass concentration of effective hydrogen molecules included in 200 mL of test water. It should be noted that the above- mentioned effective hydrogen molecule mass concentration D is of the microgram order, however, both the numerator and the denominator may be multiplied by 1000 to give:

$$D = C \cdot 1000/100 \cdot 1000 (\mu\text{g/L})$$

$$= C \cdot 10^{-5} \text{ (mg/L)} \qquad \text{(Equation 6)}$$

Then from the relationship in Equation. 2, since the hydrogen molecule mole count C of Equation 6 may be replaced with the total amount of methylene blue B, it may be established that:

$$D = N \cdot A (\text{m}\mu\text{mol}) \cdot 10^{-5} \text{ (mg/L)} \qquad \text{(Equation 7)}$$

From this Equation 7, it may be understood that the effective hydrogen molecule mass concentration D (mg/L) included in the test water may be found by multiplying the methylene blue mole concentration by volume (μmol/L) by the total amount (mL) of methylene blue solution added to reach the equivalence point.

However, the test water not only includes the hydrogen molecules (hydrogen gas) tested in the quantitative analysis here, but also includes various types of ions, oxygen molecules (oxygen gas), carbon dioxide (carbon dioxide gas), and the like. Of these, to give exemplary substance names involved in the oxidation/ reduction reaction occurring in the test water, oxygen molecules, hypochlorite, hypochlorous acid, etc. may be given besides the hydrogen molecules. Including the oxidation/ reduction reaction, such oxygen molecules, etc., normally act as the main oxidizing agent, and except for certain special cases, do not act as the reducing agent. In particular, in the test where methylene blue such as that described here is reduced, the oxygen molecules, etc. act as an oxidizing agent, and instead of reducing the methylene blue, act to oxidize the reduced methylene blue changing it to oxidized methylene blue. In other words, even if the methylene blue reduced by the activation of the molecular hydrogen either remains reduced methylene blue and clear, or remains a white deposit, in the case where it exists together with the oxygen molecule, etc, the reduced methylene blue ends up being oxidized again and returning to the original oxidized methylene blue. In addition, even if not through the methylene blue, since the activated hydrogen molecule and the oxygen molecule directly react and take an equivalent amount of the reducing power of the hydrogen molecule, this equivalent amount of methylene cannot be reduced. In other words, as shown in FIGS. 21 and 22, in the case where the oxygen molecules, etc. also exist in the hydrogen- dissolved water, an amount of hydrogen molecules equivalent to these amounts is consumed, and the total amount of methylene blue added until the equivalence point also becomes reduced in accordance with the amount of oxide.

In light of this, it may be said that the dissolved hydrogen concentration measured through quantitative analysis using methylene blue is the effective dissolved hydrogen concentration minus that consumed by oxidizing agents such as dissolved oxygen.

(F) Disclosure of reference examples and working examples

(Reference example 17)

Using alkali electrolyzed water that has been subjected to continuous electrolysis processing using electrolysis conditions of electrolysis range "4" at normal water level with a "Mini Water" electrolyzed water generation apparatus (equipped with an active charcoal filter) manufactured by MiZ Co., Ltd. as the test water, 1 mL of one-fortieth strength Pt standard solution that has been subjected to the nitrogen gas replacement described above is injected into the test water holding compartment using a syringe. This is then sufficiently stirred and mixed, and thereafter while visually observing the color change of the test water, a 1 g/L concentration (mole concentration by volume: 2677.4 μM) of methylene blue solution is added a little at a time to this test water using a syringe. The total amount of methylene blue injected until reaching the equivalence point was 1 mL, and the measured dissolved hydrogen concentration DH found by replacing the values in Equation 7 was 0.03 (mg/L). For the test water according to this working example 17, the pH, oxidation/ reduction potential ORP (mV), electric conductance EC (mS/m), water temperature T (°C), dissolved oxygen concentration DO (mg/L), measured dissolved hydrogen concentration DH (mg/L), and the measured dissolved hydrogen concentration DH (mg/L) found by replacing the values in Equation 7 are shown in Table 3, and the measured value and the effective value of DH are shown in FIG. 23. It should be noted that the types of instruments used to measure each physical property are the same as those described above.

(Reference example 18)

Using test water that consists of purified water processed by passing Fujisawa city water through an ion exchange column manufactured by Organo Corporation, boiled, and then subjected to hydrogen gas bubbling processing while allowing the temperature to cool to 20°C, 1 mL of one-fortieth strength Pt standard solution that has undergone the nitrogen gas replacement described above is injected into 200 mL of this test water in a test water holding compartment using a syringe. This is then sufficiently stirred and mixed, and thereafter while visually observing the color change of the test water, a 10 g/L concentration (mole concentration by volume: 26773.8 μM) of methylene blue solution is injected a little bit at a time into the test water using a syringe. The total amount of methylene blue solution injected until reaching the equivalence point was 6.2 mL, and the measured dissolved hydrogen concentration DH found by replacing the values in Equation 7 was 1.66 (mg/L). Each physical property value of the test water according to this reference example 18 is shown in Table 3, and the actual measured value and effective value of the dissolved hydrogen concentration DH are shown in FIG. 23.

(Working example 20)

Using electrolyzed water as test water, which is base water 6.86 of the above- mentioned sample i that has been subjected to electrolysis processing using a continuous flow method under conditions of a 1 L/min flow and 5A constant current, 1 mL of one-fortieth strength Pt standard solution that has undergone the nitrogen gas replacement described above is injected to 200 mL of this test water in a test water holding compartment using a syringe. This is then sufficiently stirred and mixed, and thereafter while visually observing the color change of the test water, a 10 g/L concentration (mole concentration by volume: 26773.8 μM) of methylene blue solution is injected a little bit at a time into the test water using a syringe. The total amount of methylene blue solution injected until reaching the equivalence point was 5.9 mL, and the measured dissolved hydrogen concentration DH found by replacing the values in Equation 7 was 1.58 (mg/L). Each physical property value of the test water according to this working example 20 is shown in Table 3, and the actual measured value and effective value of the dissolved hydrogen concentration DH are shown in FIG. 23.

(Working example 21)

Using electrolyzed water as test water, which is base water 9.18 of the above- mentioned sample v that has been subjected to electrolysis processing using a continuous flow method under conditions of a 1 L/min flow and 5A constant current, 1 mL of one-fortieth strength Pt standard solution that has undergone the nitrogen gas replacement described above is injected to 200 mL of this test water in a test water holding compartment using a syringe. This is then sufficiently stirred and mixed, and thereafter while visually observing the color change of the test water, a 10 g/L concentration (mole concentration by volume: 26773.8 μM) of methylene blue solution is injected a little bit at a time into the test water using a syringe. The total amount of methylene blue solution injected until reaching the equivalence point was 5.0 mL, and the measured dissolved hydrogen concentration DH found by replacing the values in Equation 7 was 1.34 (mg/L). Each physical property value of the test water according to this working example 21 is shown in Table 3, and the actual measured value and effective value of the dissolved hydrogen concentration DH are shown in FIG. 23.

(Working example 22)

Using electrolyzed water as test water, which is a pH buffer solution of standard buffer solution 4.01 (phthalate solution) manufactured by Wako Pure Chemical diluted to one-tenth strength with purified water that has been subjected to electrolysis processing using a continuous flow method under conditions of a 1 L/min flow and 5A constant current, 1 mL of one-fortieth strength Pt standard solution that has undergone the nitrogen gas replacement described above is injected into 200 mL of this test water in a test water holding compartment using a syringe. This is then sufficiently stirred and mixed, and thereafter while visually observing the color change of the test water, a 10 g/L concentration (mole concentration by volume: 26773.8 μM) of methylene blue solution is injected a little bit at a time into the test water using a syringe. The total amount of methylene blue solution injected until reaching the equivalence point was 6.3 mL, and the measured dissolved hydrogen concentration DH found by replacing the values in Equation 7 was 1.69 (mg/L). Each physical property value of the test water according to this working example 22 is shown in Table 3, and the actual measured value and effective value of the dissolved hydrogen concentration DH are shown in FIG. 23.

(Working example 23)

Using circulating electrolyzed water as test water, which is base water 6.86 of the above- mentioned sample i that has been subjected to electrolysis processing using a continuous flow circulating method (volume of circulatory water: 0.8 liters) for 3 minutes under conditions of a 1 L/min flow and 5A constant current, 1 mL of one-fortieth strength Pt standard solution that has undergone the nitrogen gas replacement described above is injected to 200 mL of this test water in a test water holding compartment using a syringe. This is then sufficiently stirred and mixed, and thereafter while visually observing the color change of the test water, a 10 g/L concentration (mole concentration by volume: 26773.8 μM) of methylene blue solution is injected a little bit at a time into the test water using a syringe. The total amount of methylene blue solution injected until reaching the equivalence point was 9.6 mL, and the measured dissolved hydrogen concentration DH found by replacing the values in Equation 7 was 2.57 (mg/L). Each physical property value of the test water according to this working example 23 is shown in Table 3, and the actual measured value and effective value of the dissolved hydrogen concentration DH are shown in FIG. 23.

(Working example 24)

Using circulating electrolyzed water as test water, which is base water 9.18 of the above- mentioned sample v that has been subjected to electrolysis processing using a continuous flow circulating method (volume of circulatory water: 0.8 liters) for 3 minutes under conditions of a 1 L/min flow and 5 □constant current, 1 mL of one-fortieth strength Pt standard solution that has undergone the nitrogen gas replacement described above is injected to 200 mL of this test water in a test water holding compartment using a syringe. This is then sufficiently stirred and mixed, and thereafter while visually observing the color change of the test water, a 10 g/L concentration (mole concentration by volume: 26773.8 μM) of methylene blue solution is injected a little bit at a time into the test water using a syringe. The total amount of methylene blue solution injected until reaching the equivalence point was 12.3 mL, and the measured dissolved hydrogen concentration DH found by replacing the values in Equation 7 was 3.29 (mg/L). Each physical property value of the test water according to this working example 24 is shown in Table 3, and the actual measured value and effective value of the dissolved hydrogen concentration DH are shown in FIG. 23.

(Working example 25)

Using circulating electrolyzed water as test water, which is the same pH buffer solution as working example 22 that has been subjected to electrolysis processing using a continuous flow circulating method (volume of circulatory water: 0.8 liters) for 3 minutes under conditions of a 1 L/min flow and 5A constant current, 1 mL of one-fortieth strength Pt standard solution that has undergone the nitrogen gas replacement described above is injected to 200 mL of this test water in a test water holding compartment using a syringe. This is then sufficiently stirred and mixed, and thereafter while visually observing the color change of the test water, a 10 g/L concentration (mole concentration by volume: 26773.8 μM) of methylene blue solution is injected a little bit at a time into the test water using a syringe. The total amount of methylene blue solution injected until reaching the equivalence point was 12.4 mL, and the measured dissolved hydrogen concentration DH found by replacing the values in Equation 7 was 3.32 (mg/L). Each physical property value of the test water according to this working example 25 is shown in Table 3, and the actual measured value and effective value of the dissolved hydrogen concentration DH are shown in FIG. 23.

[Table 3]

| | pH | ORP [mV] | EC [mS/m] | WATER TEMP T [°C] | DO [mg/L] | DH MEASURED [mg/L] | DH EFFECTIVE [mg/L] |
|---|---|---|---|---|---|---|---|
| REFERENCE EXAMPLE 17 | 9.8 | −171 | 17 | 21.6 | 2.67 | 0.18 | 0.03 |
| REFERENCE EXAMPLE 18 | 7.2 | −623 | 99 | 21.2 | 0.02 | 1.34 | 1.66 |
| WORKING EXAMPLE 20 | 7.0 | −616 | 99 | 22.4 | 1.00 | 1.06 | 1.58 |
| WORKING EXAMPLE 21 | 9.2 | −721 | 46 | 21.6 | 1.60 | 1.03 | 1.34 |
| WORKING EXAMPLE 22 | 4.5 | −446 | 64 | 21.7 | 1.53 | 0.81 | 1.69 |
| WORKING EXAMPLE 23 | 7.1 | −650 | 98 | 22.3 | 0.44 | 1.36 | 2.57 |
| WORKING EXAMPLE 24 | 9.6 | −764 | 54 | 22.3 | 0.45 | 2.20 | 3.29 |
| WORKING EXAMPLE 25 | 4.7 | −490 | 67 | 22.3 | 0.39 | 1.69 | 3.32 |

(G) Examination of working examples

According to Table 3 and FIG. 23, it may be understood that there is commensurate correlation between the actual measured value and the effective value of the dissolved hydrogen concentration DH since when the actual measured value is high, the effective value grows higher in response thereto. In addition, compared to the dissolved hydrogen concentration DH effective value of reference example 17, the respective effective values of DH in reference example 18 and working examples 20 through 25 all showed high concentrations exceeding 1.3 (mg/L). In particular, while the molecular hydrogen saturated solvent concentration under normal temperature (20°C) and atmospheric pressure is approximately 1.6 (mg/L), which approaches that of water, the DH effective values of working examples 20 through 25 showed between 2.5 and 3.3 (mg/L), which are exceedingly high concentrations.

Therefore oxygen molecules may be thought of as being the main oxidation agent remaining in the test water since in the quantitative analysis testing of dissolved- hydrogen concentrations performed herein, water that was pretreated with activated charcoal was used (without adding a reducing agent) in all cases to scavenge the chlorine- based oxidizers such as hypochlorous acid. It should be noted that even if the oxygen molecules are temporarily scavenged with the activated charcoal, as long as there is no sort of reducing agent used, it is difficult to scavenge with only activated charcoal because oxygen quickly blends back into the water as soon as the test water hits the outside air.

Nevertheless, with the premise that the proposed antioxidation method according the present invention is used, the fact that the concentration of oxidizing material such as dissolved oxygen may be kept as low as possible while also making the dissolved hydrogen concentration as high as possible with a reducing potential water generation apparatus such as that developed by the applicants herein is important when anticipating expression of reducing activity and antioxidation activity that may be derived from the antioxidant- functioning water according to the combination of catalysts and hydrogen- dissolved water according to the present invention.

Therefore, in attempt to define the dissolved hydrogen water according to the present invention from the standpoint of the effective value of dissolved hydrogen concentration DH found using dissolved hydrogen concentration quantitative analysis that uses oxidization/ reduction pigment according to the present invention, it is preferable that the DH effective value be 1.3 or greater, furthermore, as the dissolved hydrogen concentration DH effective value becomes higher preference increases, such as in the following order: 1.4 or greater, 1.5 or greater, 1.6 or greater, 1.7 or greater, 1.8 or greater, 1.9 or greater, 2.0 or greater, 2.1 or greater, 2.2 or greater, 2.3 or greater, 2.4 or greater, 2.5 or greater, 2.6 or greater, 2.7 or greater, 2.8 or greater, 2.9 or greater, 3.0 or greater, 3.1 or greater, 3.2 or greater, and 3.3 or greater (all units are mg/L). This is because reducing activity and antioxidation activity derived from the antioxidant- functioning water according to the combination of catalysts and hydrogen- dissolved water according to the present invention may be anticipated with higher levels.

This information proposes a new quantitative analysis method of hydrogen concentration for hydrogen- dissolved water including electrolyzed water as well as a new measure of the explicit antioxidation power held by this water. In addition, with dissolved hydrogen concentration measurement using an existing dissolved hydrogen meter, handling and measurement procedures are complicated, in terms of measurement precision such measurement is also incapable of providing sufficient satisfaction, and furthermore, related costs are extremely high. However, with the dissolved hydrogen concentration quantitative analysis method according to the present invention that uses oxidization/ reduction pigment, handling and measurement procedures is relatively simple, and if the oxidation material included in the test water is scavenged, high precision is realized in terms of accuracy because it is based on the principle of performing direct, quantitative analysis through the chemical reaction of the number of molecules of molecular hydrogen with the oxidization/ reduction pigment, and moreover, the related costs are extremely low.

Description of the embodiments herein has been made to facilitate understanding of the present invention and is not intended to limit the invention in any way. Accordingly, each element disclosed in the above embodiments may include all possible design modifications and equivalents as falls within the technical scope of the invention.

More specifically, in the general description of the invention for example, the use of a hydrogen oxidizing/ reducing enzyme, hydrogenase, or precious metal colloid in the reducing potential water for antioxidation subjects such as living cells, and the use of ultraviolet light on the reducing potential water for antioxidation subjects such as silicon wafers were shown as examples for the purpose of description. However, the present invention is not limited to such embodiments. In other words, for living cell antioxidation subjects, it is possible to use electromagnetic waves including ultraviolet light in reducing potential water, and it is possible to use a combination of electromagnetic waves including ultraviolet rays, a hydrogen oxidizing/ reducing enzyme, hydrogenase, and/or a precious metal colloid in the reducing potential water. For exemplary silicon wafer antioxidation subjects it is naturally also possible to use a hydrogen oxidizing/ reducing enzyme, hydrogenase, or precious metal colloid in the reducing potential water, and furthermore possible to use a combination of electromagnetic waves including ultraviolet rays, a hydrogen oxidizing/ reducing enzyme, hydrogenase, and/or a precious metal colloid in the reducing potential water.

Moreover, in the descriptions of the embodiments, reference examples, and working examples of the present invention, methylene blue was shown as an example of an oxidization/ reduction pigment, however, the oxidization/ reduction pigment is not limited to this. For example, new methylene blue, neutral red, indigo carmine, acid red, safranin T, phenosafranine, Capri blue, Nile blue, diphenylamine, xylenecyanol, nitrodiphenylamine, ferroin, and N-phenylanthranilic acid may also be favorably used.

Finally, a method for hydrogen recompression treatment, which is a modified example where the antioxidation method according to the present invention is applied to medical care of patients, is described. To begin with, a catalyst solution according to the present invention such as Pt colloid solution is delivered to the region of the patient's body to be subjected to treatment using a maneuver such as injection or intravenous drip. Next, the patient is placed in a recompression chamber such as that generally used for treatment of decompression sickness such as dysbarism, and the air pressure in the recompression chamber is gradually increased while observing the condition of the patient either from outside the chamber or inside the chamber. Here the gas supplied into the recompression chamber is adjusted so that hydrogen makes up between approximately 1 and 20 % of the partial pressure ratio of combined components. Then while observing the condition of the patient either from outside the chamber or inside, patient is kept in the gaseous environment that is between 2 and 3 absolute atmospheres and having an exemplary partial pressure ratio of 1:2:7 hydrogen:oxygen:nitrogen (trace amounts of other gaseous components are ignored) for approximately 1 hour, and following this, the pressure is gradually reduced to normal atmospheric pressure over a period of time equal to or longer than when pressure was being increased. Throughout this, in the region in the patient's body to be subjected to treatment (antioxidation subject), the hydrogen included in the biological fluid (hydrogen- dissolved water) via the pulmonary respiration and cutaneous respiration of the patient and the delivered catalyst meet at the subject region allowing electrons to be universally applied in the subject region. Medicinal benefits in the subject region may be anticipated through this hydrogen recompression treatment method.

## Claims

**1.** An antioxidation method, comprising:

transforming an antioxidation subject that is in an oxidation state due to a deficiency of electrons, or for which protection from oxidation is desired, into a reduced state where electrons are satisfied, by promoting the breaking reaction of molecular hydrogen used as a substrate included in hydrogen-dissolved water into a product of active hydrogen via a process employing a catalyst on the hydrogen- dissolved water.

**2.** The antioxidation method set forth in claim 1, wherein

said hydrogen- dissolved water is all water that contains hydrogen and includes either alkaline electrolyzed water generated on the cathode side when raw water is subjected to electrolysis processing between an anode and a cathode via a membrane, or water processed through bubbling or pressurized filling of hydrogen into raw water.

**3.** The antioxidation method set forth in claim 1, wherein

said hydrogen- dissolved water is a reducing potential water where the ORP is a negative value, and the ORP value corresponding to the pH shows a value that is lower than the value according to the Nernst equation or ORP = -59 pH - 80 (mV).

**4.** The antioxidation method set forth in any of claims 1 through 3, wherein

at least one reducing agent selected from the group consisting of sulfite, thiosulfate, ascorbic acid, and ascorbate is added as required to said hydrogen- dissolved water.

**5.** The antioxidation method set forth in any of claims 1 through 4, wherein

said catalyst is a precious metal colloid or a hydrogen oxidization/reduction enzyme that catalyzes the breaking reaction of molecular hydrogen used as a substrate that is included in said hydrogen- dissolved water, into a product of active hydrogen.

**6.** The antioxidation method set forth in claim 5, wherein

said hydrogen oxidization/ reduction enzyme is a hydrogenase.

**7.** The antioxidation method set forth in any of claims 1 through 4, wherein

said catalyst is one of the electromagnetic waves selected from a group consisting of visible light, ultraviolet light, and electron beams.

**8.** The antioxidation method set forth in claim 7, wherein

said electromagnetic waves are electromagnetic waves having a wavelength of approximately 300 nm or shorter.

**9.** The antioxidation method set forth in any of claims 1 through 8, wherein

said oxidation subject is either in an oxidation state due to a deficiency of electrons or a general subject for which protection from oxidation is desired, and includes living cells and to-be-rinsed subjects in industrial fields such as industrial cleaning, food cleaning, or precision cleaning.

**10.** An antioxidant- functioning water, **characterized by** comprising:

hydrogen- dissolved water to which is added a precious metal colloid or a hydrogen oxidization/ reduction enzyme that catalyzes the breaking reaction of molecular hydrogen used as a substrate that includes hydrogen-dissolved water, into a product of active hydrogen.

**11.** An antioxidant- functioning water, **characterized by** comprising:

hydrogen- dissolved water to which is added a hydrogenase that catalyzes the breaking reaction of molecular hydrogen used as a substrate that includes hydrogen- dissolved water into a product of active hydrogen.

**12.** The antioxidant- functioning water set forth in either claim 10 or claim 11, wherein

processing or manipulation for adjusting the reaction time of the catalyst is employed on said catalyst.

**13.** The antioxidant- functioning water set forth in any of claims 10 through 12, wherein

said hydrogen- dissolved water, which is all water that contains hydrogen, includes alkaline electrolyzed water generated on the cathode side when raw water is subjected to electrolysis processing between an anode and a cathode via a membrane, or water processed through bubbling or pressurized filling of hydrogen into raw water.

**14.** The antioxidant- functioning water set forth in any of claims 10 through 12, wherein

said hydrogen- dissolved water is a reducing potential water where the ORP is a negative value, and the ORP value corresponding to the pH shows a value that lower than the value according to the Nernst equation or ORP = - 59 pH - 80 (mV).

**15.** The antioxidant- functioning water set forth in any of claims 10 through 14, wherein

at least one reducing agent selected from the group consisting of sulfite, thiosulfate, ascorbic acid, and ascorbate is added as required to said hydrogen- dissolved water.

**16.** A hydrogen oxidization/ reduction enzyme, **characterized by** being prepared to allow usage in the anti-oxidation method set forth in claim 5.

**17.** A precious metal colloid, **characterized by** being prepared to allow usage in the anti-oxidation method set forth in claim 5.

**18.** A hydrogenase, **characterized by** being prepared to allow usage in the anti-oxidation method set forth in claim 6.

**19.** A rinsing method, comprising:

rinsing a to-be-rinsed item in an antioxidizing environment using the antioxidation method set forth in any one of claims 5 through 8.

**20.** A rinsing system, comprising:

rinsing a to-be-rinsed item in an antioxidizing environment using the antioxidation method set forth in any one of claims 5 through 8.

**21.** A living organism- applicable fluid, **characterized by** being prepared using the antioxidant- functioning water set forth in any of claims 10 through 14 as a main component so as to allow usage on living organisms for purposes including drinking, injection, intravenous drip, dialysis, and rinsing.

FIG. 1

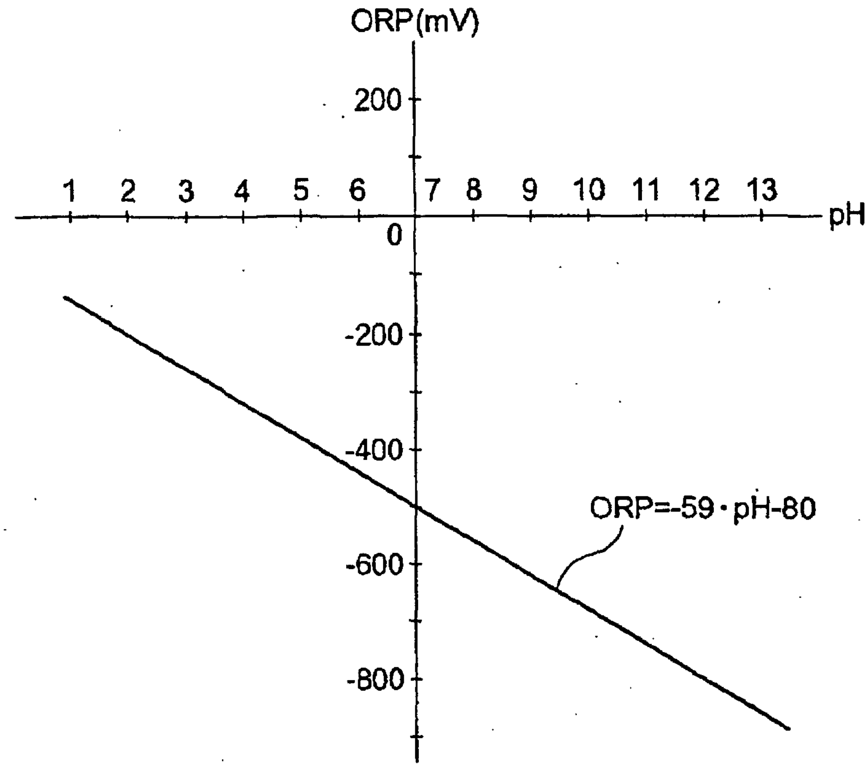
ORP(mV)
200
1 2 3 4 5 6 7 8 9 10 11 12 13
pH
0
-200
-400
-600
-800
ORP=-59·pH-80

FIG. 2

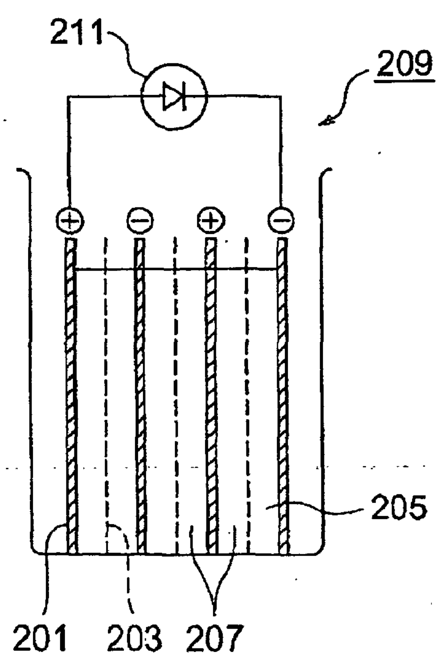
211
209
205
201
203
207

FIG. 3

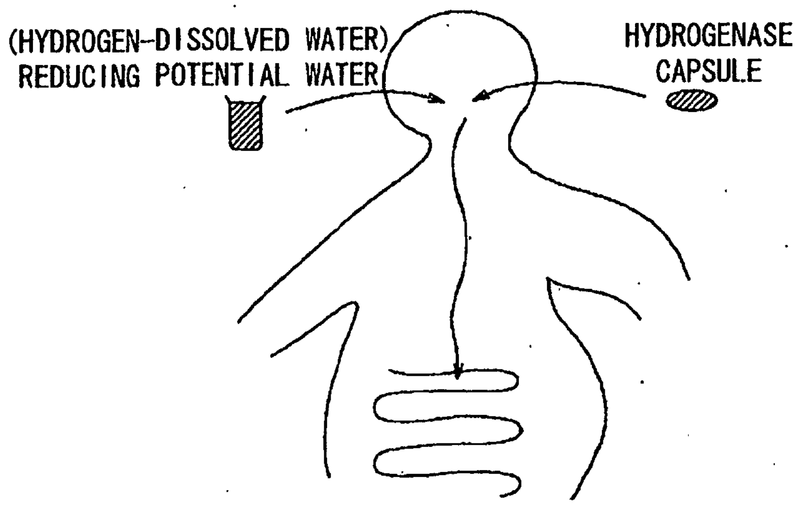
(HYDROGEN-DISSOLVED WATER)
REDUCING POTENTIAL WATER
HYDROGENASE
CAPSULE

FIG. 4

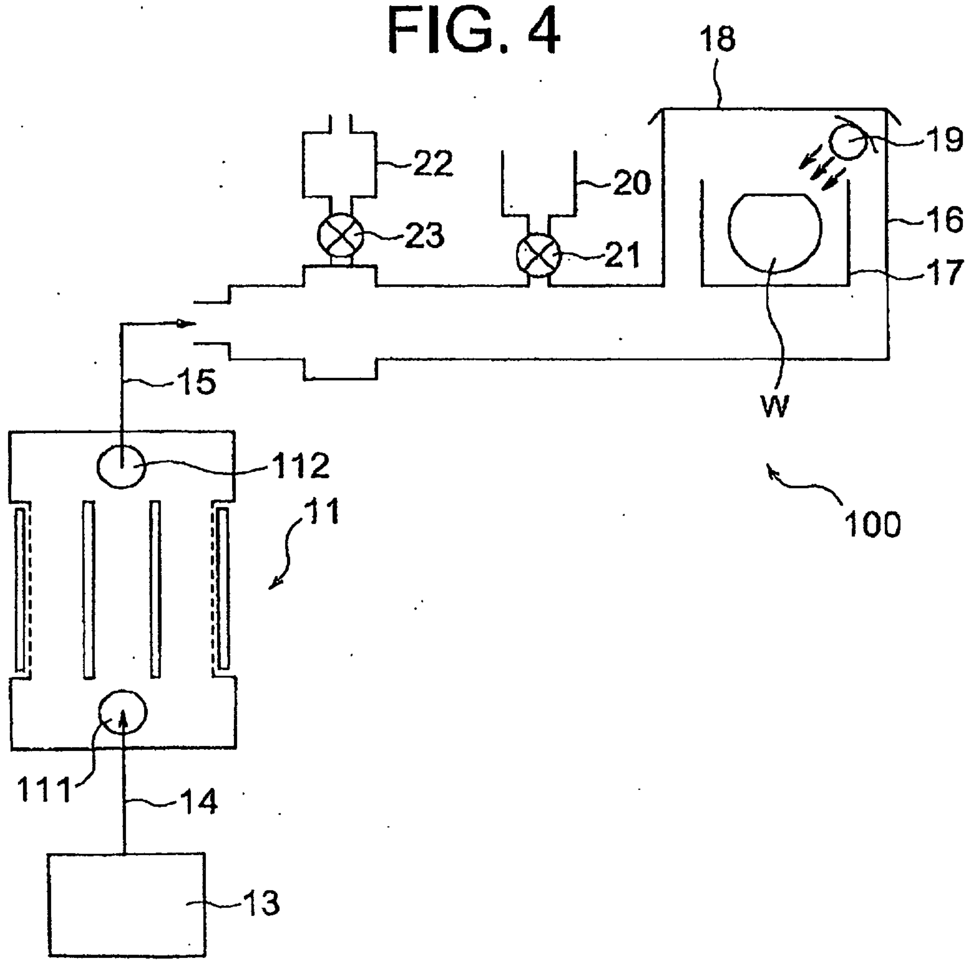
18
22
20
19
23
21
16
17
15
W
112
11
100
111
14
13

FIG.5

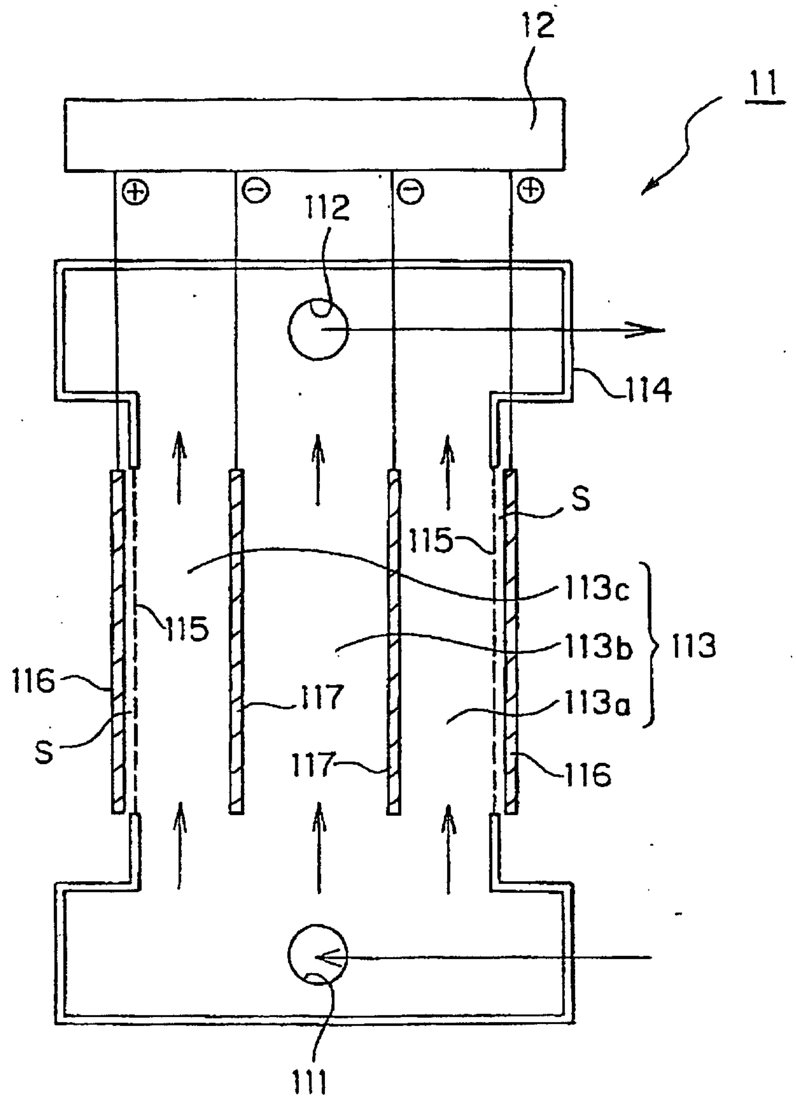

FIG. 6

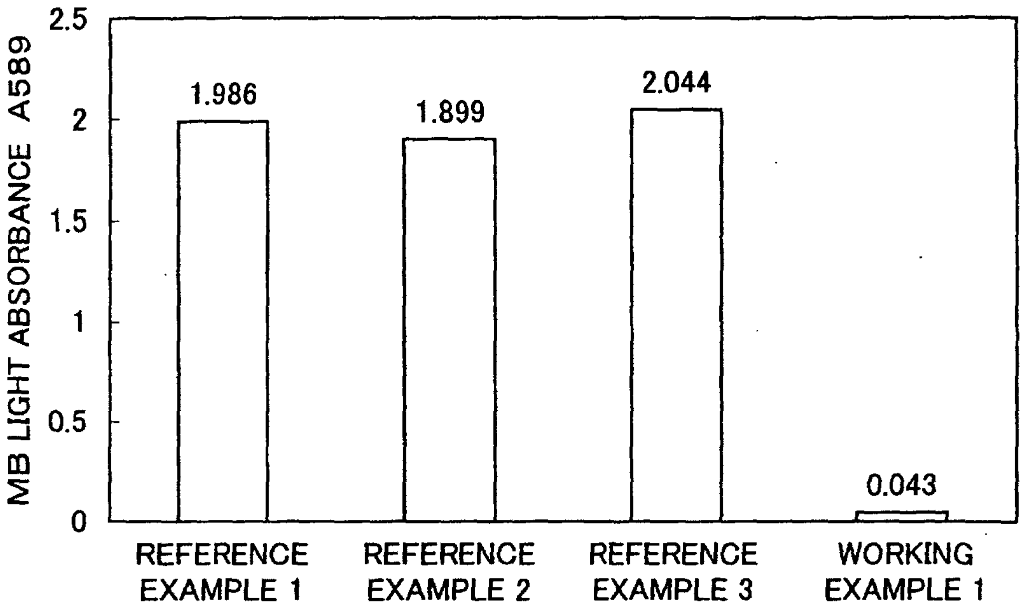
MB LIGHT ABSORBANCE A589
2.5
2
1.5
1
0.5
0
1.986
1.899
2.044
0.043
REFERENCE EXAMPLE 1
REFERENCE EXAMPLE 2
REFERENCE EXAMPLE 3
WORKING EXAMPLE 1

FIG. 7

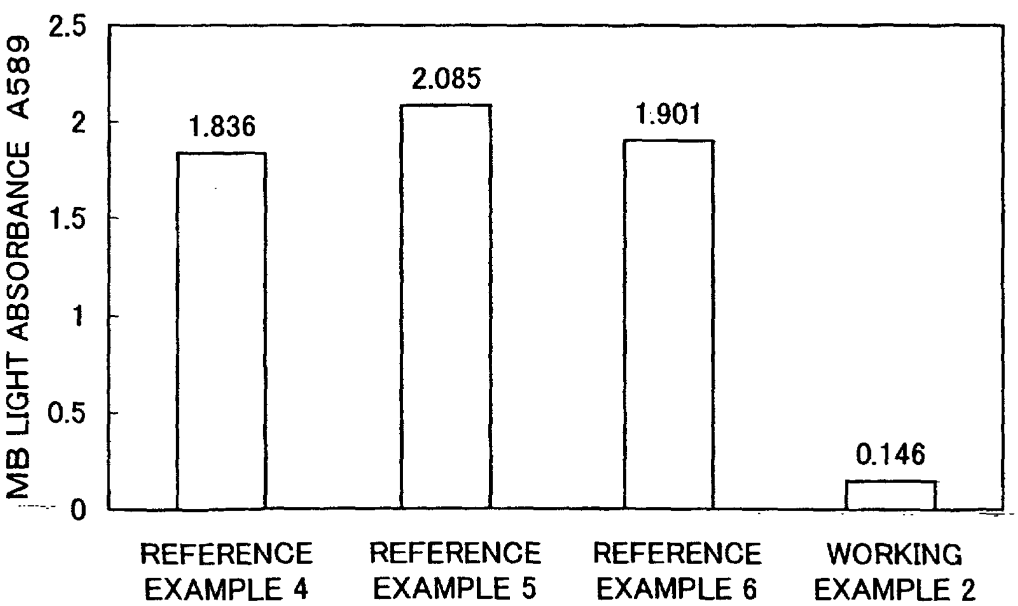
MB LIGHT ABSORBANCE A589
2.5
2
1.5
1
0.5
0
1.836
2.085
1.901
0.146
REFERENCE EXAMPLE 4
REFERENCE EXAMPLE 5
REFERENCE EXAMPLE 6
WORKING EXAMPLE 2

FIG. 8

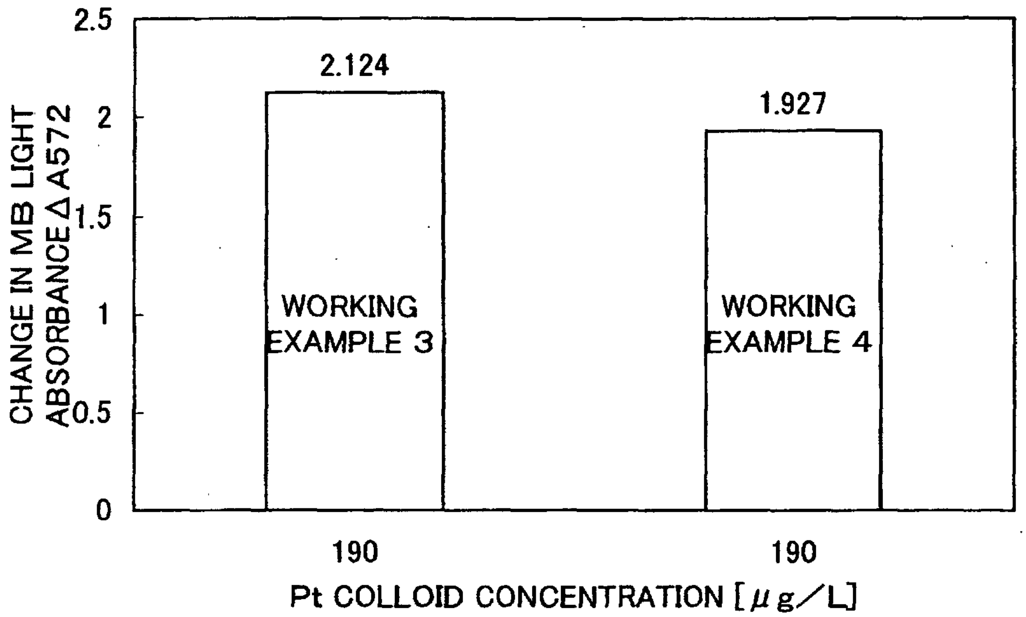
2.5
2
1.5
1
0.5
0
CHANGE IN MB LIGHT ABSORBANCE ΔA572
2.124
1.927
WORKING EXAMPLE 3
WORKING EXAMPLE 4
190
190
Pt COLLOID CONCENTRATION [μg/L]

FIG. 9

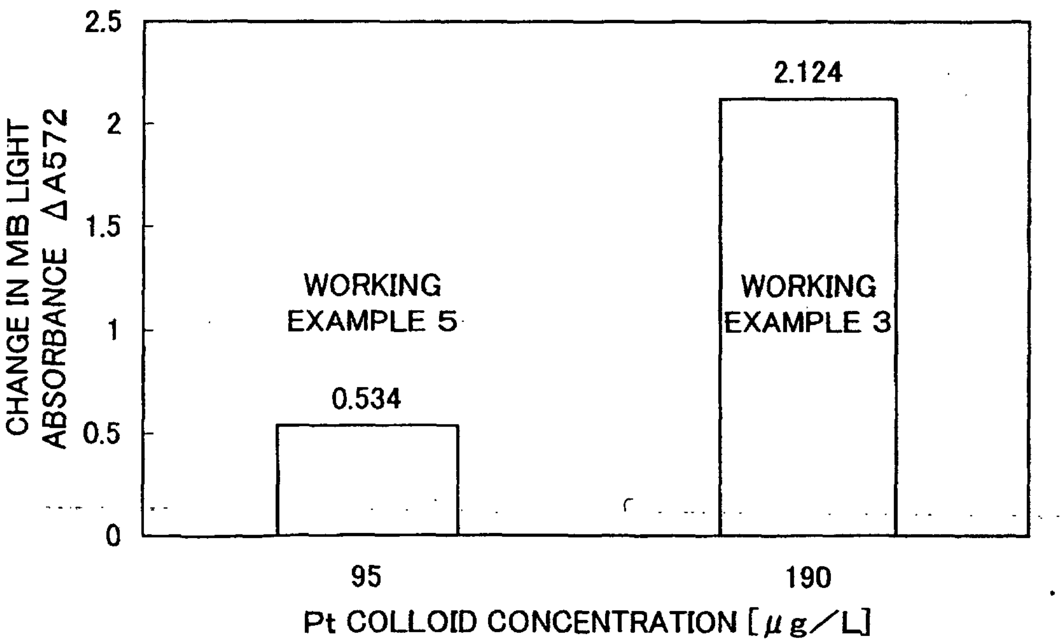
2.5
2
1.5
1
0.5
0
CHANGE IN MB LIGHT ABSORBANCE ΔA572
0.534
2.124
WORKING EXAMPLE 5
WORKING EXAMPLE 3
95
190
Pt COLLOID CONCENTRATION [μg/L]

FIG. 10

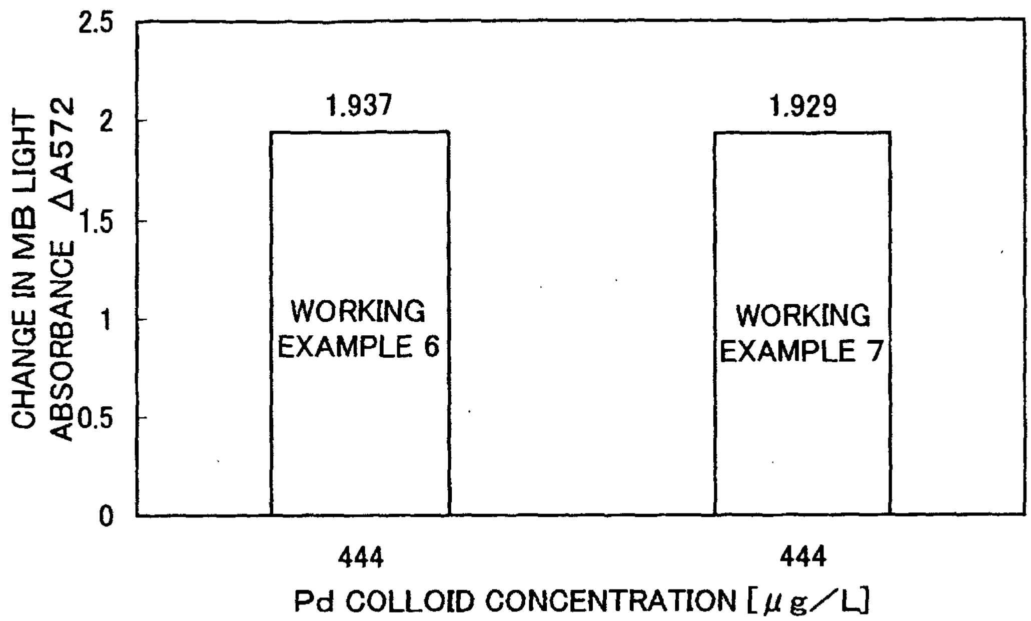
2.5
2
1.5
1
0.5
0
CHANGE IN MB LIGHT ABSORBANCE ΔA572
1.937
1.929
WORKING EXAMPLE 6
WORKING EXAMPLE 7
444
444
Pd COLLOID CONCENTRATION [μg/L]

FIG. 11

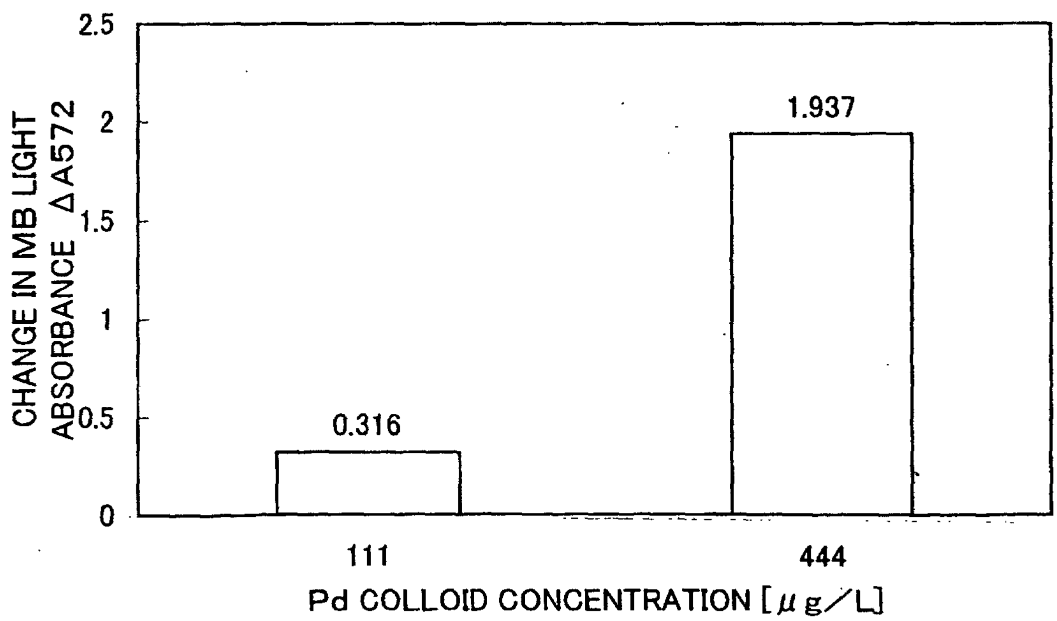
2.5
2
1.5
1
0.5
0
CHANGE IN MB LIGHT ABSORBANCE ΔA572
1.937
0.316
111
444
Pd COLLOID CONCENTRATION [μg/L]

FIG. 12

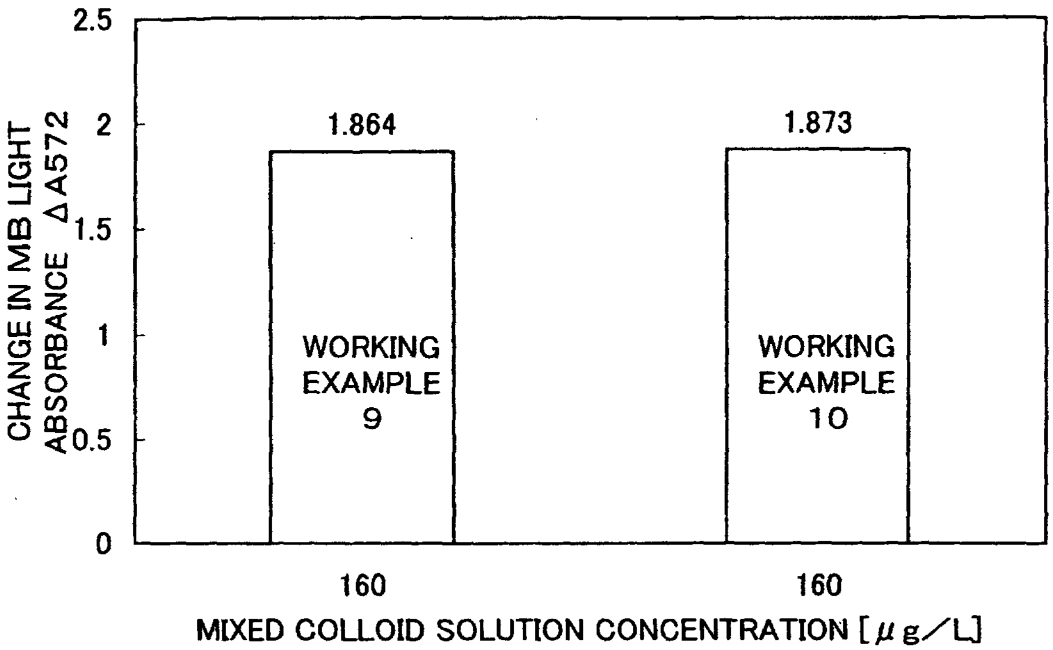
2.5
2
1.5
1
0.5
0
CHANGE IN MB LIGHT ABSORBANCE ΔA572
1.864
1.873
WORKING EXAMPLE 9
WORKING EXAMPLE 10
160
160
MIXED COLLOID SOLUTION CONCENTRATION [μg/L]

FIG. 13

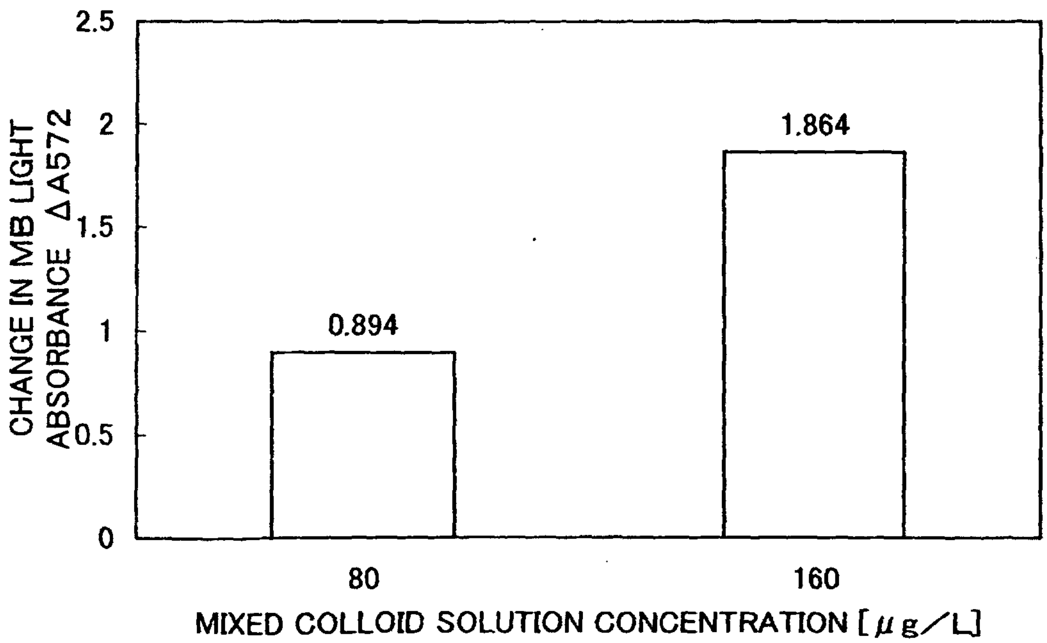
2.5
2
1.5
1
0.5
0
CHANGE IN MB LIGHT ABSORBANCE ΔA572
0.894
1.864
80
160
MIXED COLLOID SOLUTION CONCENTRATION [μg/L]

FIG. 14

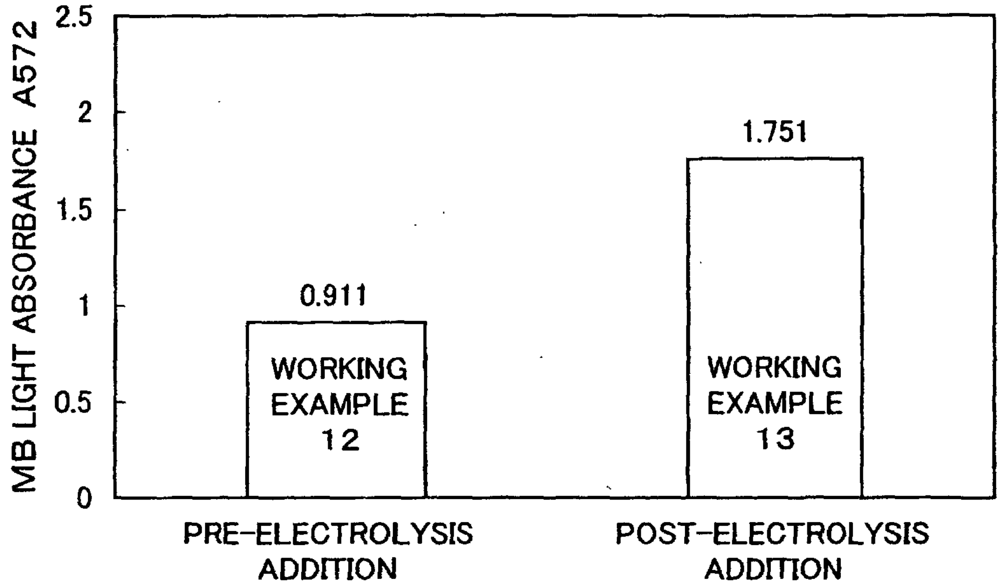
MB LIGHT ABSORBANCE A572
2.5
2
1.5
1
0.5
0
1.751
0.911
WORKING EXAMPLE 12
WORKING EXAMPLE 13
PRE-ELECTROLYSIS ADDITION
POST-ELECTROLYSIS ADDITION

FIG. 15

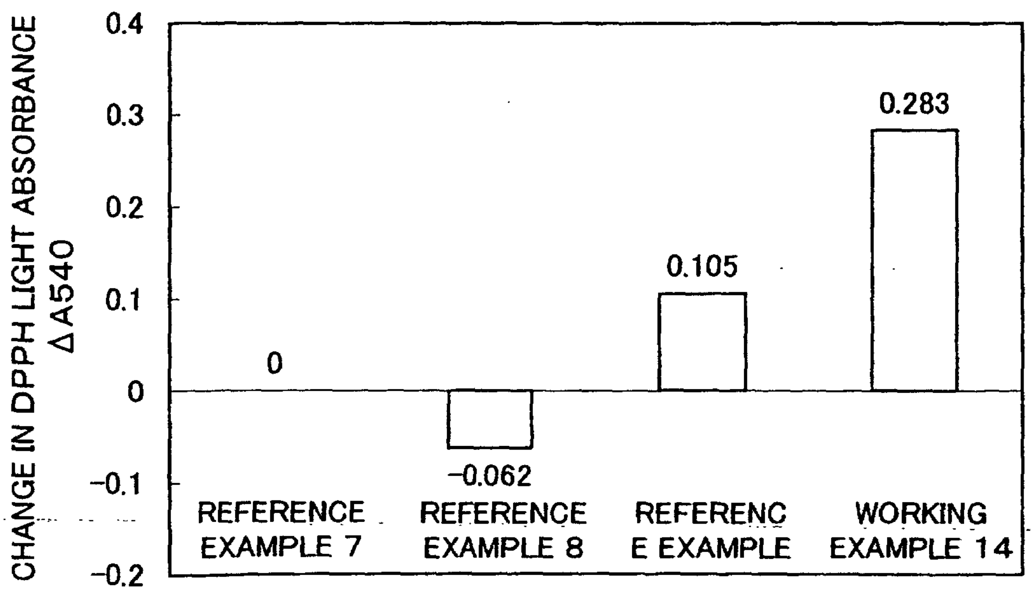
CHANGE IN DPPH LIGHT ABSORBANCE
Δ A540
0.4
0.3
0.2
0.1
0
-0.1
-0.2
0
-0.062
0.105
0.283
REFERENCE EXAMPLE 7
REFERENCE EXAMPLE 8
REFERENC E EXAMPLE
WORKING EXAMPLE 14

FIG. 16

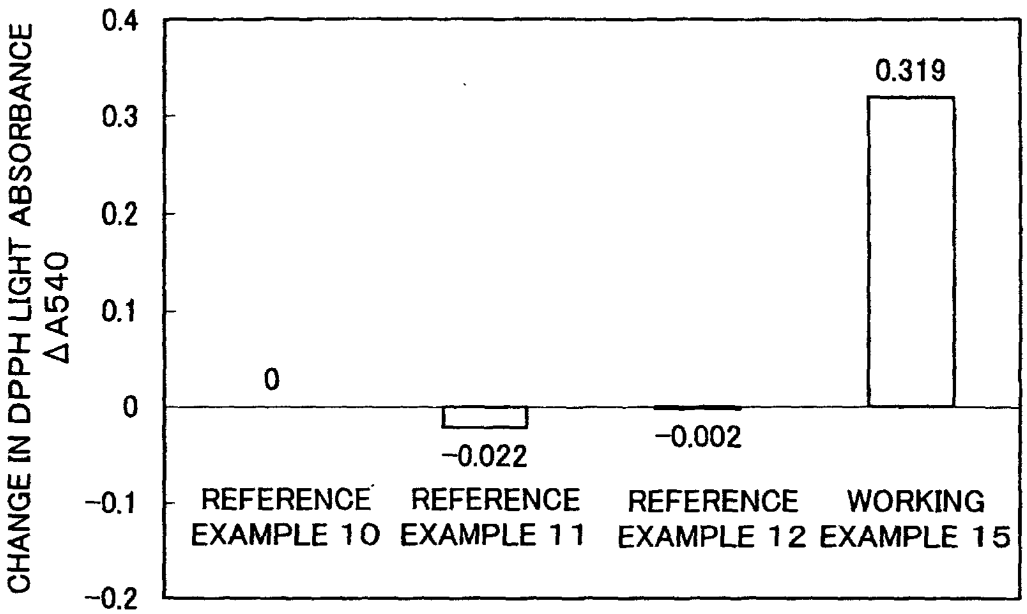
CHANGE IN DPPH LIGHT ABSORBANCE ΔA540
0.4
0.3
0.2
0.1
0
-0.1
-0.2
0.319
0
-0.022
-0.002
REFERENCE EXAMPLE 10
REFERENCE EXAMPLE 11
REFERENCE EXAMPLE 12
WORKING EXAMPLE 15

FIG. 17

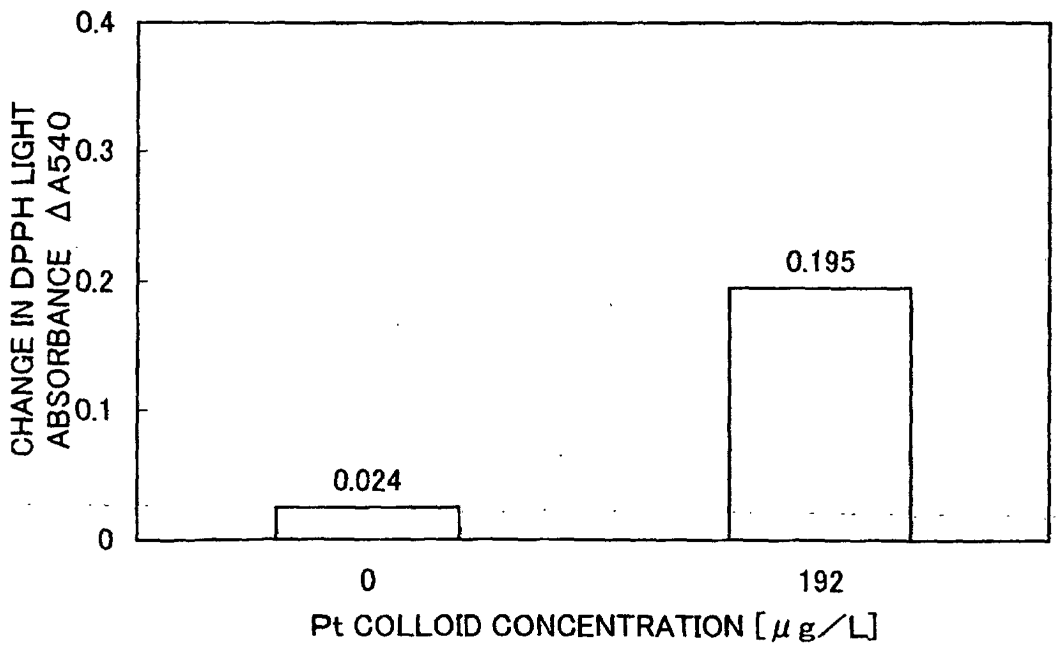
CHANGE IN DPPH LIGHT ABSORBANCE ΔA540
0.4
0.3
0.2
0.1
0
0.195
0.024
0
192
Pt COLLOID CONCENTRATION [μg/L]

FIG. 18

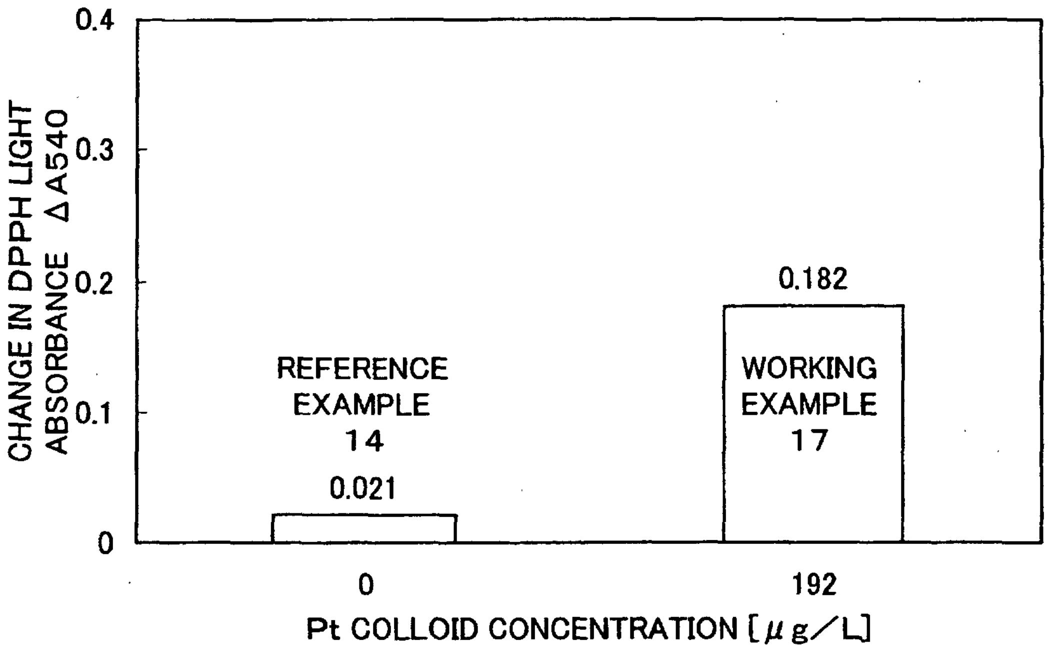
0.4
0.3
0.2
0.1
0
CHANGE IN DPPH LIGHT ABSORBANCE ΔA540
REFERENCE EXAMPLE 14
0.021
WORKING EXAMPLE 17
0.182
0
192
Pt COLLOID CONCENTRATION [μg/L]

FIG. 19

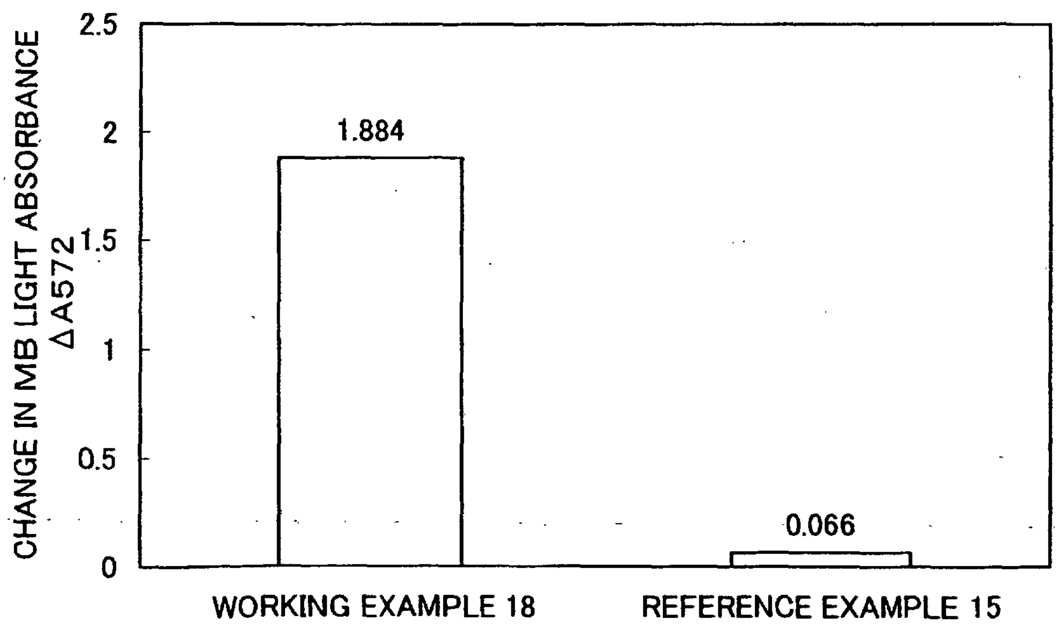
2.5
2
1.5
1
0.5
0
CHANGE IN MB LIGHT ABSORBANCE ΔA572
1.884
0.066
WORKING EXAMPLE 18
REFERENCE EXAMPLE 15

FIG. 20

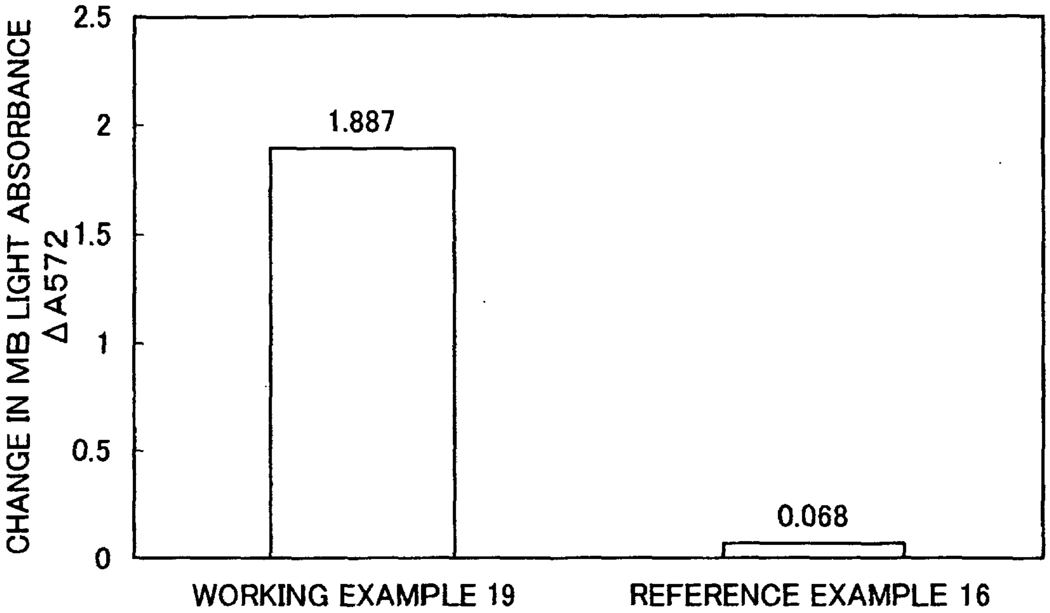
CHANGE IN MB LIGHT ABSORBANCE
ΔA572
2.5
2
1.5
1
0.5
0
1.887
0.068
WORKING EXAMPLE 19
REFERENCE EXAMPLE 16

FIG. 21

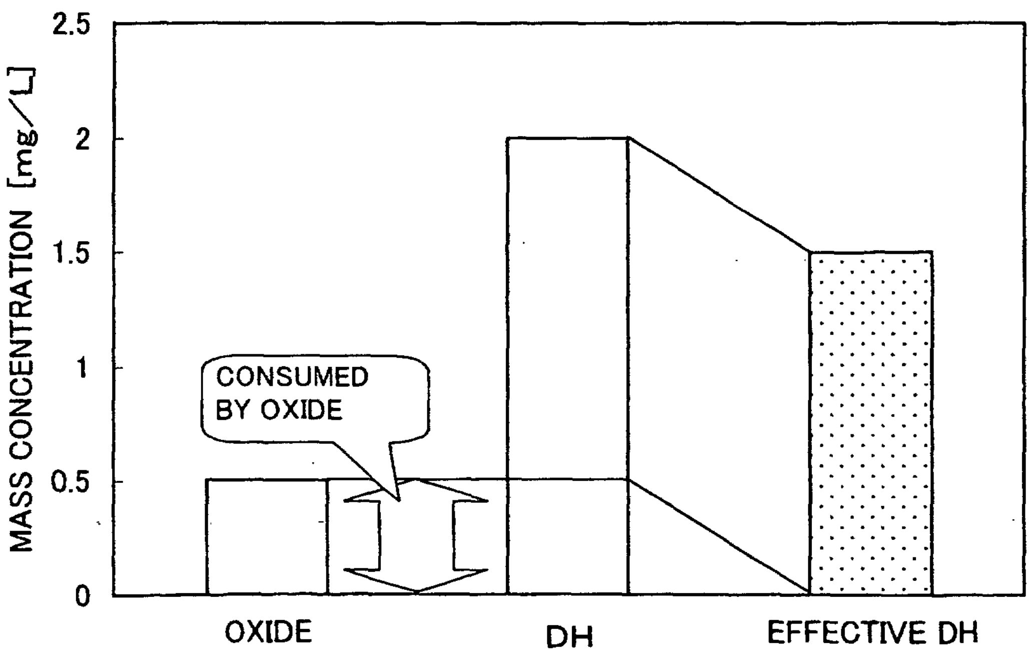
MASS CONCENTRATION [mg/L]
2.5
2
1.5
1
0.5
0
CONSUMED
BY OXIDE
OXIDE
DH
EFFECTIVE DH

FIG. 22

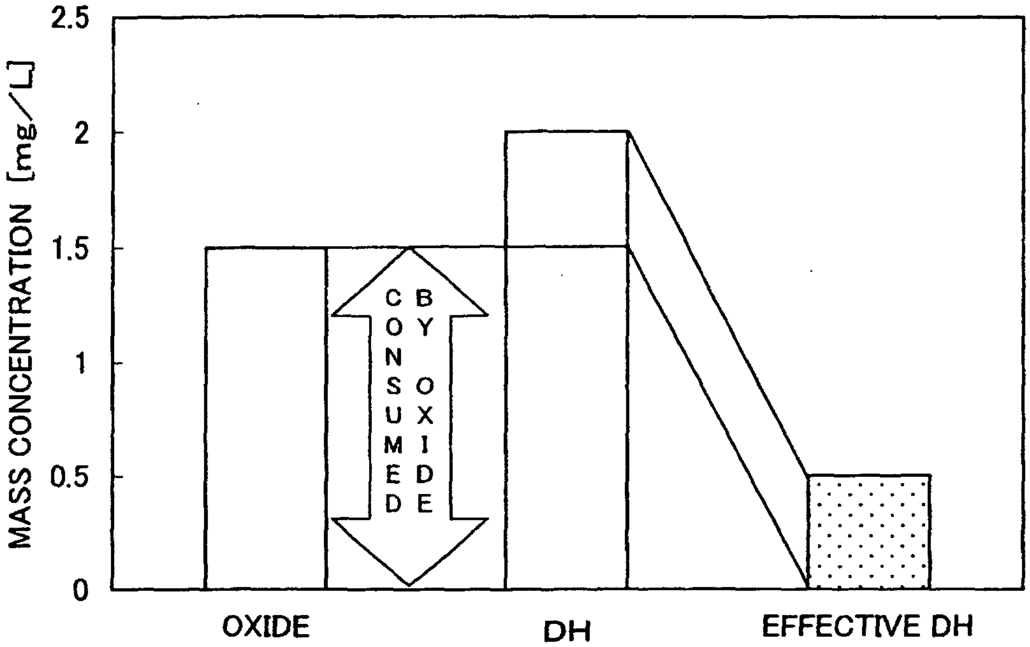
2.5
2
1.5
1
0.5
0
MASS CONCENTRATION [mg/L]
CONSUMED BY OXIDE
OXIDE
DH
EFFECTIVE DH

FIG. 23

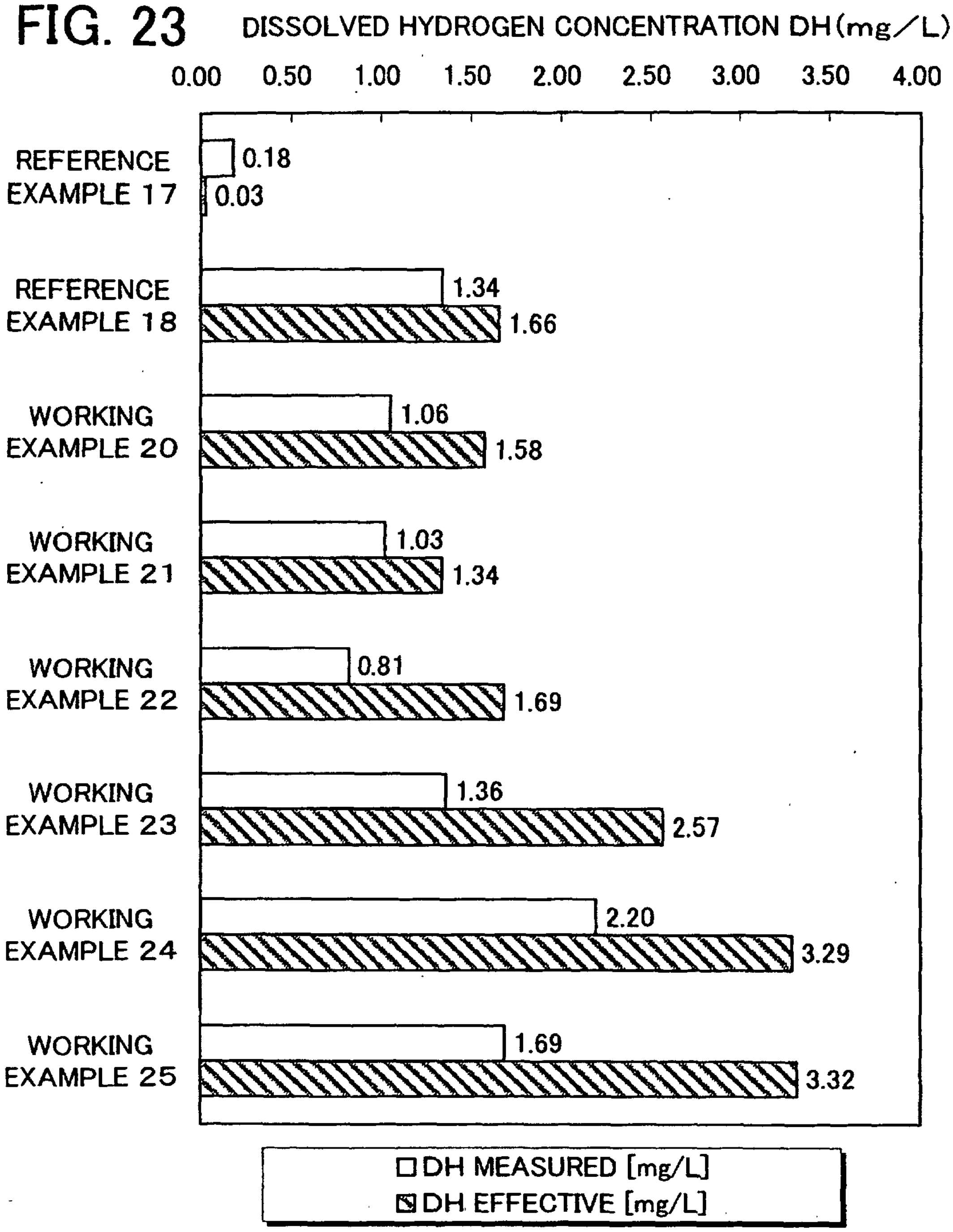
DISSOLVED HYDROGEN CONCENTRATION DH(mg/L)
0.00
0.50
1.00
1.50
2.00
2.50
3.00
3.50
4.00
REFERENCE EXAMPLE 17
0.18
0.03
REFERENCE EXAMPLE 18
1.34
1.66
WORKING EXAMPLE 20
1.06
1.58
WORKING EXAMPLE 21
1.03
1.34
WORKING EXAMPLE 22
0.81
1.69
WORKING EXAMPLE 23
1.36
2.57
WORKING EXAMPLE 24
2.20
3.29
WORKING EXAMPLE 25
1.69
3.32
DH MEASURED [mg/L]
DH EFFECTIVE [mg/L]

## INTERNATIONAL SEARCH REPORT

International PCT/JP02/05733
PCT/JP02/06560

**A. CLASSIFICATION OF SUBJECT MATTER**
Int.Cl[7] C02F1/70, C02F1/30, C02F1/32, B08B3/08, H01L21/304, C12N9/08

According to International Patent Classification (IPC) or to both national classification and IPC

**B. FIELDS SEARCHED**

Minimum documentation searched (classification system followed by classification symbols)
Int.Cl[7] C02F1/00, A61K31/28, B01J23/38, B08B3/08, H01L21/304, C12N9/08

Documentation searched other than minimum documentation to the extent that such documents are included in the fields searched

| | | | |
|---|---|---|---|
| Jitsuyo Shinan Koho | 1926-1996 | Toroku Jitsuyo Shinan Koho | 1994-2002 |
| Kokai Jitsuyo Shinan Koho | 1971-2002 | Jitsuyo Shinan Toroku Koho | 1996-2002 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br><br><br>Y | JP 4-058528 A (Ebara Research Co., Ltd.), 25 February, 1992 (25.02.92), Claims; page 2, upper right column, line 15 to lower left column, line 11; example 1 (Family: none) | 1-4,7-9, 19,20<br><br>2 |
| Y | JP 10-225664 A (Organo Corp.), 25 August, 1998 (25.08.98), Claims; page 5, Par. No. [0021]; Fig. 1 (Family: none) | 2 |
| X | JP 2001-070944 A (Matsushita Electric Works, Ltd.), 21 March, 2001 (21.03.01), Claims; page 2, Par. No. [0005] to page 3, Par. No. [0007]; page 8, Par. No. [0058] to page 9, Par. No. [0061]; Page 10, Par. Nos. [0066] to [0070] (Family: none) | 1-4,5,9,10, 12-16,21 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27 September, 2002 (27.09.02) | 15 October, 2002 (15.10.02) |
| Name and mailing address of the ISA/<br>Japanese Patent Office | Authorized officer |
| Facsimile No. | Telephone No. |

Form PCT/ISA/210 (second sheet) (July 1998)

INTERNATIONAL SEARCH REPORT

International PCT/JP02/05733
PCT/JP02/06560

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 5-317867 A (Organo Corp.), 03 December, 1993 (03.12.93), Claims; page 2, Par. No. [0008]; page 3, Par. No. [0011] (Family: none) | 1-4,9<br>5,10,12-15, 17,21 |
| Y | JP 57-081836 A (Hitachi, Ltd.), 22 May, 1982 (22.05.82), Page 2, upper left column, line 5 to lower left column, line 9 (Family: none) | 5,10,12-15, 17,21 |
| X | JP 7-184667 A (Fuji Oil Co., Ltd.), 25 July, 1995 (25.07.95), Full text (Family: none) | 1-6,9, 10-16,18 |
| X | JP 2001-010954 A (Otsuka Sangyo Kabushiki Kaisha), 16 January, 2001 (16.01.01), Claims; page 3, Par. No. [0006] to page 6, Par. No. [0008] (Family: none) | 1,3-5,9, 10,12,14, 15,17,21 |
| A | US 5403450 A (Mobitec Molecular Biologische Technologie GmbH.), 04 April, 1995 (04.04.95), Claims<br>& JP 6-500258 A & WO 92/5117 A1<br>& EP 550455 A1 & DE 4030448 A1 | 1-6,9-16, 18-21 |

Form PCT/ISA/210 (continuation of second sheet) (July 1998)